# FASCICULE DE BREVET EUROPEEN

(11) **EP 1 686 966 B1**
(45) Date de publication et mention de la délivrance du brevet: **25.01.2012**
(21) Numéro de dépôt: 04805433.2
(22) Date de dépôt: 10.11.2004
(51) Int. Cl.: A61K 9/22

(54) **COMPRIMES FAIBLEMENT DOSES A RESEAU DE POLYMERES**
NIEDRIGDOSIERTE TABLETTEN MIT EINEM POLYMERNETZ
LOW-DOSE TABLETS HAVING A NETWORK OF POLYMERS

(30) Priorité: 10.11.2003 FR 0313188
(43) Date de publication de la demande: 09.08.2006
(73) Titulaire: ETHYPHARM, 92210 St Cloud (FR)
(72) Inventeur: TCHORELOFF, Pierre, 91440 Bures sur Yvette (FR); LECLERC, Bernard, 91430 Igny (FR); BENOIST, Guillaume, 45400 Fleury les Aubrais (FR); BERTOCCHI, Laurent, 27240 Sylvains Les Moulins (FR)
(74) Mandataire: Ahner, Francis
(86) Numéro de dépôt international: PCT/FR2004/002890
(87) Numéro de publication internationale: WO 2005/046647

(56) Documents cités:
- EP-A- 0 361 874
- US-A- 5 783 215
- US-A- 6 077 533
- US-A1- 2003 017 210

## Description

La présente invention se rapporte à des comprimés administrables par voie orale, destinés à la délivrance de principes actifs, et en particulier de principes actifs faiblement dosés, permettant une libération modifiée de ces principes actifs.

Les comprimés selon l'invention sont obtenus par compression directe de microgranules constitués d'un support neutre sur lequel est appliquée une couche polymérique, sur laquelle est appliquée une couche active comportant au moins un principe actif.

Ainsi le principe actif se situe dans la couche active, mais pas dans la couche polymérique, et cette couche polymerique est intercalée entre le support neutre et la couche active.

La présente invention se rapporte également au procédé d'obtention desdits comprimés, ainsi qu'à l'utilisation qui peut en être faite pour l'administration par voie orale de médicaments, et notamment de médicaments faiblement dosés.

### Support neutre

On entend par "support neutre" ou "noyau neutre" ou encore plus simplement "neutre", des supports inertes sphériques ou quasi-sphériques de taille comprise entre 50 µm et 3 mm et préférentiellement entre 100 et 1000 µm tels que ceux habituellement utilisés dans l'industrie pharmaceutique comme support de base de principes actifs pour la constitution de microgranules par exemple.

Dans la présente invention, on utilisera préférentiellement comme support neutre des microsphéres constituées de saccharose et d'amidon de maïs. Ces microsphéres utilisées en routine dans l'industrie pharmaceutique, sont définies dans la pharmacopée européenne sous le terme *"sugar spheres*", et répondent en particulier aux spécifications suivantes : ne pas contenir plus de 92 % de saccharose calculé sur la base de la masse sèche, la masse restante étant constituée d'amidon de maïs.

### Compression directe

Le terme "compression directe" s'emploie pour qualifier l'étape d'un procédé d'obtention de comprimés consistant à comprimer "directement" le mélange d'excipients, c'est a dire sans avoir fait subir à ce mélange une transformation préalable à la compression, comme une transformation de type granulation par exemple.

Un tel procédé de compression directe n'est possible que si le mélange à comprimer présente des qualités convenables en terme .de granulométrie, de tassement, d'écoulement, et des propriétés mécaniques adaptées. Lorsque cette étape de compression directe n'est pas envisageable pour les raisons citées ci-dessus, on doit avoir recours à une étape préalable de granulation par exemple, qui modifie la texture du système granulaire, donc la granulométrie et donc le comportement en compression du mélange à comprimer.

L'étape de compression directe peut comprendre l'ajout de certains excipients favorisant l'étape de compression comme certains lubrifiants conventionnels par exemple.

### Microgranules et excipients fonctionnalisés

Les microgranules de la présente invention se rapportent à des unités galéniques sphériques, constitués en leur centre d'un support neutre, recouvert d'au moins une couche polymérique qui est elle-même recouverte d'au moins une couche contenant le principe actif. De tels microgranules sont assemblés en comprimés conformément à la présente invention par compression directe.

On parlera d'excipient fonctionnalisé pour désigner le support neutre recouvert de la couche polymérique après séchage, laquelle couche polymérique est dépourvue de principe actif. En effet, cette association particulière correspond à une fonctionalisation des excipients sur lesquels va être déposée la couche active, rendant ces excipients propices à l'obtention d'un système a libération modifiée après compression, conformément à l'invention.

### Comprimés faiblement dosés

Dans la présente demande, on entend par comprimés faiblement dosés, des comprimés dont la charge en principe actif est de faible à très faible, c'est à dire inférieure à 50 mg par comprimé, preférentiellement inférieure à 25 mg par comprimé, préférentiellement encore inférieure à 10 mg par comprimé. En particulier, les comprimés conformes à la présente invention sont adaptés à des formulations dont les dosage peuvent être de l'ordre du microgramme, dans la mesure où le procédé de préparation de ces comprimés décrit ci-après permet de travailler avec ces concentrations minimes en garantissant l'homogénéité de répartition par unité de prise.

### Désintégrants

On parlera de désintégrant ou encore d'agent de désintégration pour désigner une substance dont le rôle est d'accélérer la désintégration d'un comprimé et donc la dispersion du principe actif dans le milieu de dissolution (milieu aqueux ou sucs gastriques par exemple). Ces agents sont utilisés comme adjuvant aux formules de comprimés pour favoriser leur désagrégation et la libération du principe actif. En général, ce sont des agents extrêmement hydrophiles ayant souvent la propriété de gonfler rapidement au contact de l'eau, l'hydratation et/ou le gonflement et l'augmentation de volume provoquant la désintégration des comprimes les contenant.

### Comprimés matriciels et comprimés à réseau de polymére

On entend par comprimés matriciels, des comprimés dans lesquels le principe actif est mélangé de façon intime au polymère. Ce type de comprimés est classiquement employé pour la réalisation de comprimés à libération prolongée par exemple. Cette association étroite est responsable d'une modification du comportement du principe actif dans le milieu de dissolution. En effet le comportement de ce dernier va être fortement influencé par le comportement du polymère constitutif de la matrice en présence d'eau.

Les comprimés matriciels sont classiquement obtenus soit par simple mélange de poudres, soit par granulation afin d'améliorer les propriétés du mélange avant compression. Dans la présente demande, on parlera de comprimés matriciels pour désigner ces types de comprimés obtenus par simple mélange de poudres et d'effet matriciel" pour désigner un profil de dissolution similaire à celui obtenu à partir d'un comprimé matriciel classique.

Les comprimes conformes à l'invention ont au contraire une structure multiparticulaire car ils sont obtenus par compression directe de microgranules. Ils ont l'avantage de présenter un effet matriciel sans présenter les inconvénients des comprimés matriciels classiques.

On parlera dans la présente demande de matrice "in situ" pour qualifier le réseau polymérique formé au sein des comprimés de l'invention, suite à la compression des microgranules, et de «comprimés à réseau de polymères» pour qualifier les comprimés conformes à l'invention.

### Libération modifiée

Dans la présente demande, on utilisera le terme libération modifiée pour désigner un profil de libération du principe actif modifié par rapport à celui qu'aurait présenté le principe actif seul dans le milieu de dissolution. Dans la présente invention la modification du profil est due à l'utilisation d'un polymère dont la nature va conduire soit à une accélération de la libération de l'actif, on parlera alors de libération accélérée, soit à un retard dans la libération de l'actif, on parlera de libération retardée, soit à un prolongement de la libération de l'actif, on parlera de libération prolongée.

Le but de la présente invention est d'offrir une nouvelle composition phamaceutique orale à base de comprimés présentant un effet matriciel, c'est à dire un comportement dans le milieu de dissolution similaire à celui de comprimés *matriciels.* De tels comprimés permettent ainsi d'envisager soit une libération modifiée du principe actif dans l'organisme, soit une désagrégation rapide du comprimé en fonction de la nature du polymère employé, et ce, même pour de très faibles dosages en principe actifs.

La présente invention se rapporte à des comprimés obtenus par compression directe de microgranules constitués de trois parties distinctes. Ces microgranules sont ainsi constitués, du centre vers la périphérie par un support neutre, préférentiellement un noyau de sucre et d'amidon de maïs, puis par une couche polymérique intermédiaire dépourvue de principe actif comprenant au moins un polymère d'intérêt, c'est à dire un polymère dont on veut exploiter les propriétés pour influer sur le profil de libération du principe actif et enfin d'une couche active comprenant le ou les principes actifs proprement dits.

### Les formes à libération prolongée

Les formes pharmaceutiques solides a libération prolongée peuvent être classées en deux catégories majeures : les formes dites "réservoir" dans lesquelles le principe actif est maintenu à l'intérieur d'un compartiment délimité par une membrane ralentissant sa diffusion, et les formes dites "matricielles" dans lesquelles le principe actif est mélangé de manière homogène au sein d'une matrice polymérique ralentissant ou accélérant sa diffusion.

Les comprimés à libération prolongée de type réservoirs sont généralement constitués d'un mélange contenant le principe actif recouvert d'un film diffusionnel prolongeant la libération de l'actif dans le milieu de dissolution. On parle alors de comprimés pelliculés. Dans ce cas, le film recouvre le comprimé de façon uniforme.

Ce type de comprimés pelliculés est classiquement employé dans l'industrie pharmaceutique pour modifier le profil de libération du principe actif et/ ou le protéger.

Un cas particulier concerne les comprimés à libération prolongée de type réservoirs qui peuvent être constitués de microgranules recouverts d'une couche externe régulant la diffusion du principe actif dans le milieu de dissolution. Ces microgranules sont directement soumis aux contraintes de la compression. En effet, l'obtention de comprimés suffisamment cohésifs pour être manipulés nécessite d'appliquer aux microgranules une force de compression relativement importante. Les contraintes physiques exercées sur les microgranules sont souvent responsables d'une altération de la couche externe responsable de la libération prolongée. Il en résulte alors des altérations du profil de libération, conduisant à une libération du principe actif plus rapide que celle escomptée et en tout état de cause imprévisible et donc non reproductible.

Pour pallier ces inconvénients, plusieurs compositions particulières ont été développées. Ainsi, le brevet EP 1032374 décrit une composition pharmaceutique à base de sphéroïdes déformables, dont le film extérieur résiste à la contrainte de compression en se déformant sans se rompre. Cependant, un tel système est relativement complexe et nécessite l'emploi de plusieurs polymères particuliers et notamment d'excipients thermoplastiques.

Les systèmes dits matriciel sont en revanche souvent beaucoup plus simples à fabriquer. En effet, dans de telles compositions, le principe actif est simplement mélangé au polymère, au sein d'une matrice polymérique.

Les matrices polymériques peuvent être de nature hydrophile, c'est à dire constituées de polymères de grande affinité pour l'eau. De telles matrices assurent généralement la libération prolongée du principe actif en formant, au contact du milieu aqueux environnant, un gel qui, par sa cinétique de gonflement, d'érosion ou sa capacité à jouer un rôle de barrière diffusionnelle, prolonge la libération du principe actif dans le milieu de dissolution.

Ainsi, le brevet Nostrum Pharmaceutical US 6,416,786 décrit des comprimés à effet prolongé constitués d'une matrice hydrophile composée d'un mélange d'un hydrocolloïde tel que la gomme xanthane avec un éther de cellulose. Un tel mélange procure à la composition un effet prolongé synergique dû à l'action combinée des deux polymères. En effet, le caractère hydrophile de la gomme xanthane rend la formation du gel au contact du milieu aqueux plus rapide, l'éther de cellulose garantissant au gel un certain maintien dans le temps, la gomme xanthane utilisée seule ayant tendance à s'éroder rapidement dans le milieu de dissolution.

De même, le brevet Valentine Entreprises US 5,292,534 décrit une formulation à libération prolongée de niacine et de gomme xanthane, cette formulation pouvant se présenter sous la forme de gélules ou de comprimés. Le principe actif est mélange de façon intime la gomme xanthane.

Cependant, de tels mélanges ne sont pas adaptés à une forme faiblement dosée. En effet, le principal inconvénient des comprimés matriciels classiques fabriqués à partir d'un mélange de poudres de nature différente est que plus le rapport massique entre la poudre de polymère et la poudre de principe actif est important, plus le risque d'inhomogénéite de teneur augmente. En effet, du fait des différences de taille, de forme et de densité des particules d'excipients et des particules de principe actif, le mélange des deux poudres risque d'engendrer un phénomène de mélange imparfait ou de démélange. Le démélange correspond à la séparation des poudres de naturels différentes, il intervient notamment suite aux mouvements infligés au mélange par exemple lors du transport ou de la manipulation du mélange de poudres à l'échelle industrielle.

Ce phénomène entraîne inévitablement une inhomogénéité de répartition en principe actif au sein du mélange. Une telle inhomogénéité entraîne à l'échelle industrielle des variations de teneur en principe actif qui peuvent être très importantes d'un comprimé à l'autre et qui doivent faire l'objet d'une maîtrise toute particulière. Ce phénomène s'accentue de façon évidente lorsque la proportion de principe actif diminue dans le mélange.

En outre, un autre inconvénient souvent rencontré pour les formes matricielles comprimées est la nécessité de granuler tout ou partie des constituants avant l'étape de compression. Cette prégranulation rend le procédé plus long et plus complexe qu'une seule étape de compression directe.

Le brevet américain US 5,783,215 (D1) décrit des granules à libération contrôlée contenant un coeur inerte solide, sur lequel est appliqué une couche contenant un principe actif dispersé dans un polymère hydrophile, cette couche active étant ensuite recouverte d'une membrane polymérique permettant la libération contrôlée du principe actif.

Le brevet US 6,077,533 décrit des granules de sulfate de morphine comprenant un coeur inerte solide, sur lequel est applique par poudrage une couche active contenant du sulfate de morphine mélange intimement avec du lactose hydrate. Ces granules peuvent servir à préparer une formulation multi-particulaire de sphéroïdes de sulfate de morphine à libération immédiate ou à libération prolongée. Ces granules sont recouverts d'une couche permettant la libération prolongée du principe actif.

La demande américaine US 2003/017210 décrit des microgranules de cisplatine à libération contrôlée comprenant un microgranule immédiat contenant du cisplatine, enrobé d'une couche d'un agent enrobant. L'agent enrobant permet de modifier la libération du cisplatine et se compose de préférence de polymère, tel que les polymères cellulosiques ou les Copolymères diacide méthacrylique.

La demande de brevet européenne EP 361 874 décrit des granules ayant un noyau, sur lequel est appliqué une dispersion d'hydroxypropylcellulose faiblement substitué (L-HPC). Cette dispersion peut en outre comprendre un principe d'actif ou d'autres additifs, autres que le L-HPC, uniformément dispersés ou dissous.

### Les formes à désintégration rapide

Les comprimés à désintégration rapide de l'art antérieur sont généralement constitués à partir le mélange d'excipients et du principe actif, lorsque la compression directe de ce mélange s'y prête ou à partir de grains contenant le principe actif, obtenus après granulation par exemple, puis comprimés, après mélange d'une phase externe, comprenant l'agent délitant lorsque la compression directe n'est pas envisageable.

Ainsi, dans le brevet EP 548356 des granules enrobés de principes actifs sont assemblés par compression après avoir préalablement été mélanges au sein d'une matrice de compression constituée entre autre d'un sucre de compression directe nécessaire à cette étape du procédé.

De tels comprimés ne sont pas non plus adaptés à la délivrance de principes actifs faiblement dosés. En effet, là encore se pose le problème du démélange de poudres de nature différente qui rend très délicate l'obtention de comprimés homogènes et de faible dosage.

Le brevet US 5,607,697 décrit des comprimés à désintégration rapide dépourvus de matrice de compression, dans lesquels le principe actif est simplement granulé avec un excipient de compression. Ces granules sont ensuite enrobés et comprimés. Cependant, même dans ce type de procède sans matrice, l'excipient de compression est mélangé de façon peut contrôlable au principe actif. Ce type de formulation ne permet donc pas d'assurer une homogénéité de teneur suffisante pour des principes actifs faiblement dosés.

Ainsi, l'inconvénient majeur des comprimés matriciels à libération prolongée et des comprimés classiques à désintégration rapide est qu'ils sont mal adaptés comme support de principes actifs faiblement, voire très faiblement dosées pour lesquels l'homogénéité de teneur est primordiale et impérative.

Cependant, la fabrication de comprimés faiblement dosés permettant de s'affranchir de ces problèmes de démélange et d'inhomogénéité de teneur lorsque le rapport entre le principe actif et les autres excipients est très faible a déjà fait l'objet de travaux.

Ainsi, le brevet Ethypharm EP 1 200 071 décrit des comprimés faiblement dosés en principe actif, obtenus par compression directe de simples noyaux neutres ou *"sugar spheres"* sur lequel a été préalablement pulvérisée une solution de principe actif pouvant contenir un agent liant. De tels comprimés ont l'avantage de présenter une grande homogénéité de et une grande reproductibilité, même à des concentrations très basses en actif

Par ailleurs, de tels comprimés ne nécessitent pas d'excipients de compression particuliers et peuvent être soumis à une étape de compression directe. Cependant, ces comprimés ne permettant pas d'envisager une libération modifiée du principe actif et la libération de l'actif dans le milieu est immédiate à quasi-immédiate, sauf si le comprimé a été enrobé, comme c'est le cas dans les formes dites réservoir.

Ainsi, les comprimés matriciels de l'art antérieur permettant une libération modifiée du principe actif ne permettent pas d'envisager l'incorporation de principes actifs faiblement dosées, par ailleurs, les comprimés possédant cette qualité ne sont pas adaptés à une libération modifiée du principe actif.

C'est pourquoi, le problème technique que se propose de résoudre l'invention. tient dans la réalisation de comprimés présentant un effet matriciel, tout en évitant les inconvénients liés à la fabrication de ce type de comprimés, en particulier la difficulté d'obtention de comprimés matriciels, faiblement dosés présentant une teneur homogène en principe actif.

Le comportement dans l'organisme du principe actif contenu dans de tels comprimés va ainsi dépendre de la nature du ou des polymères constituant la matrice polymérique ou le réseau polymérique.

### Avantages des comprimés de la présente invention

La présente invention présente plusieurs avantages par rapport aux formulations de l'art antérieur.

Tout d'abord, les comprimés conformes à l'invention sont de structure relativement simple et peuvent être mis en oeuvre facilement dans l'industrie pharmaceutique par un procédé simple, reproductible, relativement peu coûteux et de fort rendement à toutes les étapes de fabrication comme cela est décrit ci-après.

D'une part, le procédé selon l'invention ne comporte aucune étape de granulation on de pré-granulation nécessaire à la compression de la plupart des mélanges d'excipients sous forme de poudre.

D'autre part, l'étape de compression directe proprement dite est avantageusement réalisée sans excipients de compression à l'exception d'un éventuel lubrifiant conventionnel utilisé en faible quantité et en tout état de cause à moins de 5% en masse de la masse finale du comprimé. Cette dernière caractéristique est due notamment à l'excellent comportement en compression des microgranules constituant les comprimés conformes à l'invention.

Un autre avantage de l'invention est qu'elle permet la fabrication de comprimés à libération prolongée ou à désintégration rapide à partir d'une quantité très faible de polymère, ce qui réduit d'autant le coût du procédé et le temps nécessaire à sa mise en oeuvre.

Le procédé conforme à l'invention est en outre extrêmement reproductible et permet donc d'envisager la fabrication industrielle de tels comprimés en satisfaisant aux exigences réglementaires de l'industrie pharmaceutique en matière de qualité et de conformité du médicament, notamment en ce qui concerne l'homogénéité de masse et de teneur des comprimés, comme cela est montré dans le tableau 7. Ce tableau montre également la qualité du procédé en terme de rendement puisque pour chacun des trois lots exemplifiés, le rendement massique global est toujours supérieur à 90 %.

Un autre avantage des comprimés selon l'invention est qu'ils présentent une grande homogénéité, non seulement de forme, de taille, et de densité, mais encore de teneur en principe actif, même à des très faibles dosages, c'est à dire à des dosages inférieurs à 50 mg, préférentiellement inférieurs à 25 mg, voire à 10 mg par comprimé.

Par ailleurs, du fait de cette homogénéité structurelle, le risque de ségrégation des microgranules lors de la fabrication du comprimé est très faible, ce qui diminue d'autant les risques d'inhomogénéité de teneur entre les comprimés et même entre deux moitiés d'un comprimé sémble.

Ce dernier aspect est particulièrement important dans la mesure où les comprimés matriciels classiques ne sont généralement pas du tout adaptés à des formes sécables.

On comprend aisément que dans le cas des comprimés de type réservoirs, la forme sécable n'est pas envisageable car la continuité du film diffusionnel ne peut être rompue. Dans le cas des comprimés matriciels, et notamment des comprimés à matrice polymérique, d'une part la structure même du comprimé ne se prête pas à une coupure nette du comprimé, et d'autre part, l'homogénéité de teneur en actif entre les deux moitiés du comprimé n'est pas garantie.

Au contraire, dans les comprimés de l'invention, l'homogénéité de teneur est assurée de part la répartition précise de l'actif à la surface des microgranules neutres et ce, même à de faibles dosages. La faible taille des microgranules permet une répartition massique équilibrée et précise pour les deux moitiés du comprimé lors de la section. Les comprimés selon l'invention peuvent donc tout à fait s'envisager sous une forme sécable.

Par ailleurs, les comprimés conformes à l'invention peuvent tout à fait. être recouverts, après les étapes du procédé décrit ci-après d'une ou plusieurs couches de pelliculage supplémentaires destinées à modifier encore le profil de libération du principe actif. En effet, les comprimés conformes à l'invention sont, à l'issue du procédé de fabrication tout à fait semblables à des comprimés classiques et peuvent donc sans aucune difficulté faire l'objet d'un pelliculage adapté aux besoins. Ce pelliculage peut être fait dans le but de protéger le principe actif, de conférer un profil de libération gastro-résistant ou de masquer le goût de l'actif par exemple.

On peut par exemple prévoir de pelliculer les comprimés conformes à l'invention avec une ou plusieurs couches d'un agent de pelliculage gastro-résistant afin de limiter la libération de l'actif au niveau gastrique.

De plus, les composants nécessaires à la réalisation des comprimés conformes à l'invention sont à la fois agréés pour un usage pharmaceutique, peu coûteux, et pour ce qui concerne le polymère d'intérêt, nécessaire en relativement faible quantité comme cela est décrit par la suite.

Enfin, lesdits comprimés sont utilisables pour tous types de principes actifs, et plus particulièrement pour les principes actifs dont l'action sur l'organisme est très puissante, et qui doivent donc être administrés très faible dose est être libérés de façon progressive dans l'organisme ou au contraire de manière accélérée. Les hormones ou leurs dérives par exemple, les principes actifs agissant sur le système nerveux central ou le système cardiovasculaire, les antibiotiques, les anitiviraux, les analgésiques et les anti-inflammatoires sont particulièrement utilisables dans les comprimés de l'invention.

En outre, les comprimés de la présente invention, sont également particulièrement adaptés à l'administration de principes actifs dont la marge thérapeutique est très étroite. En effet, dans ce cas, il est d'une importance primordial de pourvoir administrer avec certitude une dose très précise de médicament. Or les excellentes qualités d'homogénéité de masse et de teneur des comprimés conformes à l'invention les rendent tout à fait aptes à ce genre d'administration délicate.

La présente invention se rapporte à des comprimés, utilisables dans l'industrie pharmaceutique pour l'administration de principes actifs, et permettant une libération modifiée. En particulier, les comprimés conformes à l'invention sont particulièrement adaptés à l'administration de principes actifs faiblement dosés.

La présente invention a pour objet des comprimés faiblement dosés obtenus par compression, directe de microgranules qui sont essentiellement constitués d'un support neutre, recouvert d'une couche polymérique comprenant au moins un polymère pharmaceutiquement acceptable et permettant la libération modifiée de principes actifs en milieu aqueux, dépourvue de principe actif, sur laquelle est appliquée une couche active comportant au moins un principe actif.

Ces comprimés présentent l'avantage de contenir le principe actif réparti de façon homogène. Ces comprimés peuvent donc se présenter sous forme sécable.

La présente invention a en outre pour objet les produits intermédiaires pouvant être utilisés pour la préparation des comprimés. Ces intermédiaires sont :
- l'excipient fonctionnalisé constitué d'un support neutre recouvert d'une couche polymérique comprenant au moins un polymère pharmaceutiquement acceptable et permettant la libération modifiée de principes actifs en milieu aqueux,
- le microgranule constitué d'un support neutre recouvert d'une couche polymérique comprenant au moins un polymère pharmaceutiquement acceptable et permettant la libération modifiée de principes actifs en milieu aqueux, sur laquelle est appliquée une couche active comportant au moins un principe actif.

Les microgranules selon l'invention ont une taille comprise entre 50 et 3000 µm environ.

La couche polymérique peut comporter en outre au moins un agent liant pharmaceutiquement acceptable. On utilisera de préférence un agent liant de nature hydrophile se dissolvant facilement dans l'eau et ou dans l'éthanol. Ainsi, on utilisera par exemple comme agent liant dans la couche polymerique la polyvinylpyrrolidone, en particulier la polyvinylpyrrolidone vendue sous le nom de marque PVP K-30^{®}.

La couche polymerique peut aussi comporter en outre une cire ou un de ses dérives, ou un dérivé d'acides gras du glycérol, ou un mélange de ceux-ci.

On pourra employer par exemple la cire d'abeille naturelle ou purifiée.

Les esters d'acides gras du glycérol et leurs dérivés peuvent être choisis parmi le glycérol monostéarate, le glycérol monooléate et le glycérol palmitostéarate par exemple, mais également des mélanges de glycérides et d'esters d'acides gras du polyéthylène glycol tels que ceux appartenant à la famille des lauroyl macrogolglycerides, vendus sous le non commercial Gelucire®.

Avantageusement, le polymère pharmaceutiquement acceptable et permettant la libération modifiée de principes actifs en milieu aqueux, qui est contenu dans la couche polymerique, représente de 1 à 100 % en masse de la masse du support neutre, de préférence 1 à 50 % en masse de la masse du support neutre.

### Le support neutre

Ledit support neutre à la base des microgranules de l'invention est de préférence une microsphére constituée de saccharose et d'amidon de maïs, de taille comprise entre 50 et 3000 µm, de préférence entre 100 et 1000 µm, et plus préférentiellement encore entre 100 et 500 µm.

Ces microsphères utilisées en routine dans l'industrie pharmaceutique sont définies dans la pharmacopée européenne sous le terme "*sugar spheres*"*.*

Ces "neutres" sont avantageusement utilisables comme support de base aux excipients fonctionnalisés cités plus haut notamment du fait de leur grande homogénéité de masse, de taille, de forme et de surface spécifique qui en font un outil de choix pour la fabrication de médicaments faiblement dosés obtenus par pulvérisation et pour lesquels on doit s'assurer de la parfaite homogénéité des lots en matière de teneur en principe actif.

Par ailleurs, comme cela été montré dans le brevet EP 1 200 071, les neutres sont des excipients de choix pour les systèmes comprimés. En particulier, les "*sugar spheres*" ont montré un excellent comportement en compression directe, d'autant plus intéressant que ces neutres peuvent être comprimés sans autres excipients de compression, a l'exception d'un lubrifiant en très faible quantité.

### Les polymères d'intérêt

L'effet "matriciel" observé sur les comprimés conformes à l'invention, c'est à dire leur propension à se comporter comme des comprimés matriciels conventionnels dans lesquels on aurait mélangé de façon homogène polymère et principe actifs, est du à l'étape de compression qui génère une structure particulière en permettant de façon inattendue la création d'une matrice *"in situ*". Une telle matrice se forme en effet seulement au moment de la compression.

Dans une telle configuration, le principe actif se trouve alors emprisonné dans un véritable réseau de polymère constitue par les différentes couches polymériques des microgranules adjacents.

En effet, un réseau de polymère qui sera préférentiellement continu pour des comprimés à libération prolongée se forme autour du principe actif lors de la compression.

Ainsi, à partir d'une certaine concentration seuil on polymère, la déformation des grains de polymères déposes à la surface des microgranules neutres suite à la contrainte d'écrasement exercée par les forces de compression, peut permettre la création d'un réseau de polymère. La compression contribue à la déformation et à l'élongation des grains de polymère qui finissent par rentrer en contact de façon intime les uns avec les autres, créant ainsi un véritable réseau observable seulement à partir d'une quantité critique de polymère, correspondant à la concentration seuil.

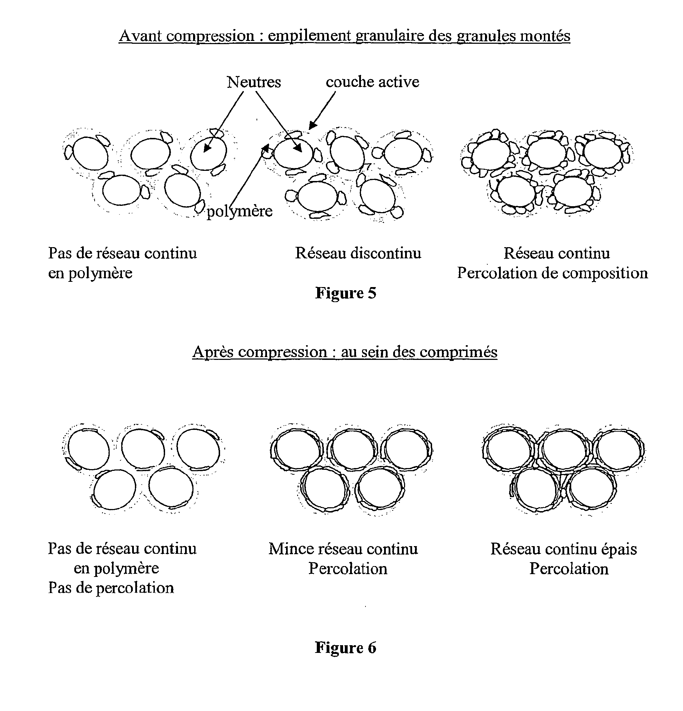

Dans le cas de l'utilisation de polymères destinés a prolonger le profil de libération de l'actif, la concentration seuil nécessaire a la création d'une matrice "in situ" est atteinte dès lors qu'on forme un réseau dit "réseau de percolation" correspondant à un réseau continu de particules de polymère après l'étape de compression. La formation d'un tel réseau entre les microgranules est responsable d'une libération prolongée du principe actif pris au coeur de cet ensemble. Les schémas de la figure 5 permettent une bonne compréhension du phénomène d'aplatissement et de création du réseau continu suite à l'étape de compression.

Dans le cas de l'utilisation de polymères désintégrants, l'obtention d'un réseau de percolation n'est pas nécessaire, car l'effet matriciel est observé dès lors qu'un réseau, même discontinu de particules de polymère se forme à l'issue de l'étape de compression. Un tel réseau discontinu suffit à assurer la pénétration rapide de l'eau au sein du comprimé ainsi formé, du fait de la forte affinité pour l'eau des particules de polymère désintégrant. Cette hydratation conduisant à un gonflement brusque et donc à une désintégration rapide du comprimé après son contact avec le milieu aqueux.

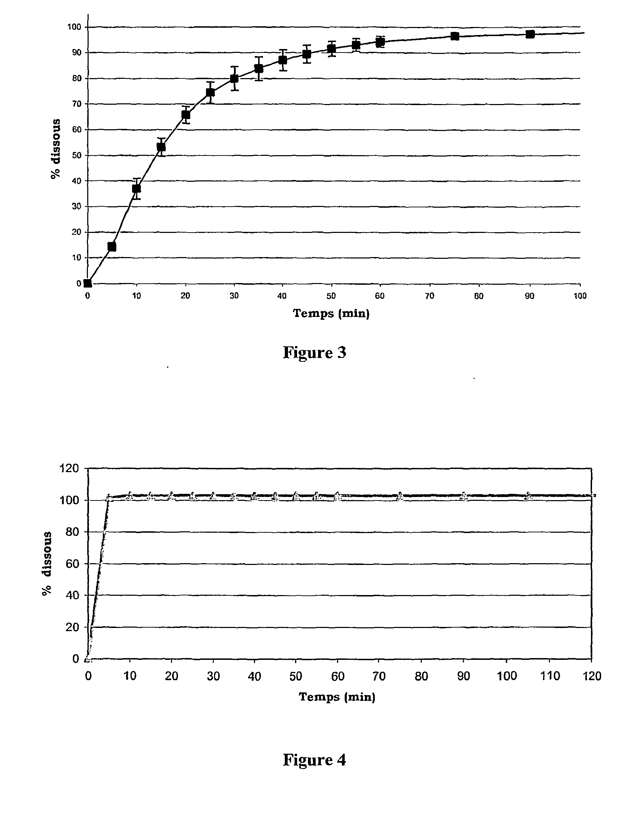

En effet, contrairement au cas des granules vrac (voir figure 4), une fois la compression effectuée, chaque couche de principe actif, se trouve en contact avec la couche d'actif du microgranule voisin. Cette double couche d'actif va ainsi se trouver enserrée entre deux couches de polymère, excepté sur la surface extérieure du comprimé. On parle alors de structure "en sandwich".

Ainsi le polymère d'intérêt reste particulièrement bien focalisé non seulement par rapport au principe actif, car le contact polymère / actif est très important de part la surface développée, mais également à l'échelle du comprimé : la structure et la composition du comprimé étant très homogènes. On obtient ainsi des comprimés constitués de multiples couches d'actif, chacune étant prise en sandwich entre deux couches du polymère choisi. Par conséquent, à l'échelle du comprimé, les composants sont idéalement positionnés : la double couche d'actif se situant prise en sandwich entre deux couches de polymère modifiant la libération de l'actif lors du contact avec le milieux de dissolution.

Ainsi, si on emploie comme polymère d'intérêt un polymère utilisé de façon classique pour la constitution de comprimés matriciels à libération prolongée, on va obtenir un comprimé à libération prolongée.

Si au contraire on emploie comme polymère un excipient utilisé de façon classique pour la désintégration de comprimés, on va obtenir un comprimé à désintégration rapide permettant une dissolution rapide du principe actif dans le milieu de dissolution, ce d'autant plus facilement que l'excipient support ("sugar spheres") est hydrophile et très soluble dans l'eau.

Le comportement du comprimé lors de son contact avec le milieu de dissolution va donc être fonction du comportement du polymère formant ce réseau ou cette matrice.

On comprend ainsi les multiples avantages de l'invention qui découlent du fait de pouvoir adopter de façon extrêmement simple le polymère à l'application envisagée. En effet, les microgranules neutres qui forment le support des comprimés conformes à l'invention conservent leur bon comportement en compression quel que soit le type de polymère employé déposé à leur surface, même si ces propriétés peuvent se voir modifier dans certains cas et devront être ajustées par le jeu de la formulation. D'autre part, les comprimés selon l'invention permettent de travailler avec une large gamme tant de polymère que de principes actifs, tout en garantissant une parfaite homogénéité de teneur en principe actif, et en particulier à de très faibles dosages.

Le polymère contenu dans la couche polymérique des microgranules constituant les comprimés objet de la présente invention est de préférence choisi parmi les polymères à libération prolongée et les polymères désintégrants.

### • Polymères désintégrants

Les polymères utilisés dans la présente invention pour favoriser la désintégration du comprimé, et donc la dispersion rapide du principe actif dans le milieu de dissolution sont des polymères généralement employés pour leur capacité à gonfler rapidement au contact de l'eau conduisant à un éclatement du comprimé les contenant.

Les polymères désintégrants sont avantageusement choisis parmi les dérivés de la polyvinylpyrrolidone, les dérivés de l'amidon, les sels de calcium et de magnésium et les dérivés de la carboxymethylcellulose, ainsi que leurs mélanges.

Les dérivés de la polyvinylpyrrolidone peuvent être choisis parmi la crospovidone ou la povidone.

Les polymères dérivés de l'amidon peuvent être choisis parmi le carboxymethylamidon sodique tel que celui vendu sous la marque Explotab^{®} par exemple ou encore l'amidon réticulé.

Les dérivées de la cellulose peuvent être choisis parmi la carboxyméthylcelluose sodique ou la croscarmellose sodique telle que celle vendue sous la marque Ac-Di-Sol^{®} par exemple, la méthylcellulose ou l'hydroxypropylcellulose faiblement substituée par exemple.

Tous ces polymères peuvent être montés par poudrage à la surface des microgranules neutres de la même façon que pour les polymères à libération prolongée comme cela est décrit en détail dans le procédé ci-dessous.

### • Polymères pour libération prolongée

Pour ce qui est des polymères de nature hydrophile, on choisira avantageusement des polymères à propriétés gélifiantes et d'une façon préférentielle, on choisira des polymères d'une viscosité supérieure à 1000 mPa.s (milli Pascal seconde) mesurée dans une solution aqueuse à 2 % m/m à 20°C, selon les pharmacopées européennes ou américaines.

Les polymères à propriétés gélifiantes ont la propriété de former rapidement au contact du milieu aqueux un gel visqueux par gonflement, encore appelé hydrogel, qui prolonge la libération du principe actif dans ce milieu. Les polymères très hydrophiles, présentant des capacités d'hydratation rapides et donc de gonflement rapide avec formation d'un gel, seront préférentiellement utilisés car ils conduisent à un mode de libération diffusionnel.

En effet, on considère que trois mécanismes majeurs sont responsables de la dégradation du gel et donc de la libération prolongée de l'actif contenu dans le gel vers le milieu de dissolution : un phénomène de gonflement, un phénomène de diffusion et un phénomène d'érosion.

Le gonflement est l'étape de formation de l'hydrogel et correspond à l'hydratation du polymère. La vitesse de gonflement conditionne donc la libération de l'actif. En effet, tant que l'hydrogel n'est pas formé, l'actif ne peut être libéré, ni par érosion, ni par diffusion. La cinétique de gonflement est donc un facteur limitant dans la libération de l'actif.

L'érosion étant un phénomène relativement peu prévisible, il est préférable pour la libération prolongée de médicament d'avoir recours à un système de libération majoritairement diffusionnel. Ainsi, les polyméres hydrophiles à propriétés gélifiantes sont-ils de bons candidats pour ce type ce systèmes. On peut en effet ajuster, suivant la densité et l'épaisseur du gel formé en milieu aqueux la vitesse de libération du principe actif.

Les polymères à libération prolongée de nature hydrophile sont de préférence choisis parmi les polymères dérivés de la cellulose, les polysaccharides naturels ou naturels modifiés tels que les gommes, les galactomananes, les glucomananes, les succinoglycanes ou les scléroglucanes, les carboméres et les poly(oxyde d'ethylène) ainsi que leurs mélanges.

On utilisera par exemple les polymères dérivés de la cellulose et en particulier les dérivés semi-synthétiques du groupe des éthers de cellulose de moyenne à forte viscosité tels que ceux utilisés de façon classique pour la constitution de matrices hydrophiles prolongeant la libération du principe actif.

Les dérivés de la cellulose peuvent être choisis parmi l'hydroxyéthylcellulose (HEC), l'hydroxypropylcellulose (HPC), et l'hydroxypropylméthylcellulose (HPMC).

Les carbomères pouvant être utilisés sont par exemple ceux dont le nom commercial est Carbopol^{®} 971P, Carbopol^{®} 974P, éventuellement Carbopol^{®} 934P.

Les polysaccharides naturels ou naturels modifiés peuvent être choisis parmi les gommes et les galactomananes, les glucomananes, les succinoglycanes et les scléroglucanes.

Les polymères appartenant au groupe des poly(oxyde d'éthylène) peuvent être choisis parmi ceux dont le nom commercial est Polyox WSR^{®}.

De manière préférentielle, les polymères de nature hydrophile présentant des propriétés gélifiantes selon l'invention appartiennent à la catégorie des gommes, en particulier des gommes d'origine naturelle ou microbienne telles que l'acide alginique, les alginates, notamment l'alginate de sodium, l'agar-agar, les carraghénanes, la gomme de caroube, la gomme guar, la gomme adragante, la gomme arabique, la gomme de cassia, la gomme xanthane, la gomme karaya, la gomme tara, la gomme tragacanthe et la gomme gelante.

D'une manière préférée, on utilisera pour la réalisation de la présente invention des gommes d'origine bactérienne et notamment la gomme xanthane.

La gomme xanthane est un polysaccharide naturel d'origine bactérienne issu de la fermentation de l'amidon de maïs par la bactérie *Xanthomonas campestris.* Ce biopolymère de haut poids moléculaire est constitué de multiples unités répétées comportant chacune cinq molécules d'oses: deux molécules de glucose, deux molécules de mannose et une molécule d'acide glucuronique. A l'état solide, la gomme xanthane se présente sous la forme d'une poudre dont les particules ont une taille comprise entre 10 et 180 µm environ et selon les grades, une forme relativement sphérique.

Avantageusement, le polymère hydrophile à propriétés gélifiantes conforme à la présente invention représente 1 à 100 % en masse de la masse du support neutre, de préférence 1 à 50 % en masse de la masse du support neutre.

Parmi les polymères de nature différente, on peut employer certains polymères et copolymères dérives de l'acide méthacrylique insolubles dans l'eau quelque soit le pH, en particulier certains copolymères de l'acide méthacrylique vendus sous le nom commercial Eudragit^{®} tels que l'Eudragit^{®} RS PO, l'Eudragit^{®} RL PO, l'Eudragit^{®} RL et l'Eudragit^{®} RS qui appartiennent à la famille des chlorures de poly(éthyl acrylate, méthyl méthacrylate, triméthylamonioéthyl méthacrylate).

On peut également utiliser certains polymères cellulosiques insolubles dans l'eau comme l'éthylcellulose et l'acétate de cellulose, ainsi que leurs mélanges.

De tels polymères conduisent à une libération prolongée de l'actif dans le milieu de dissolution essentiellement par un mécanisme d'érosion. En effet, leurs propriétés retardent la pénétration de l'eau au sein du comprimé, le principe actif se libère donc au fur et à mesure qu'il rentre en contact avec le milieu aqueux suite à l'érosion progressive de la matrice de polymère.

On peut également utiliser certains polymères muco-adhésifs comme la carboxyméthylcellulose sodique, les carbomères, l'alginate de sodium, l'hydroxyéthylcellulose, l'hydroxypropylcellulose, l'hydroxypropylméthylcellulose, la gélatine, la gomme guar, les poly(oxyde d'éthylène), la dextrine ou le chitosane,

De tels polymères sont généralement employés pour préparer des comprimés destinés à une administration sublinguale ou tansmucosale.

Tous les polymères ci-dessus décrits peuvent être montés par poudrage à la surface des microgranules neutres comme cela est décrit en détail dans le procédé ci-dessous.

### • Mélange de polymères de nature différente

Pour la réalisation de la présente invention on peut prévoir que la couche polymérique appliquée sur les neutres comprenne un mélange de polymères de différentes natures, utilisés soit en synergie pour l'obtention d'un profil de libération particulier soit en complémentarité pour modifier le profil de dissolution et apporter une qualité supplémentaire au comprimé.

Le mélange de polymères constituant la couche polymérique devra alors être adapté aux qualités recherchées pour la matrice polymérique, en particulier, la proportion de chacun des polymères devra être ajustée pour satisfaire les contraintes liées au profil de libération attendu ou aux autres fonctionnalités décrites.

### Le principe actif

La couche active des microgranules constituant les comprimés conformes à l'invention comprend un principe actif pharmaceutique qui peut être de toute nature.

La couche active peut comporter en outre au moins un agent liant pharmaceutiquement acceptable.

Pour une réalisation simple de l'invention, on travaillera avec des principes actifs soluble dans l'eau et/ou dans l'alcool ou tout autre solvant peu ou non toxique et pharmaceutiquement acceptable. Cependant, des principes actifs peu, voire très peu solubles dans ces solvants peuvent cependant être envisagés pour la réalisation de la présente invention, dans la mesure où on utilisera préférentiellement de très faibles concentrations en actif, concentrations pour lesquelles on peut envisager la dissolution de la plupart de ces actifs.

Les principes actifs agissant à très faible concentration sont particulièrement adaptés aux comprimés conformes à l'invention.

Les comprimés selon la présente invention peuvent comprendre en tant que principe actif, les hormones ou leurs dérivés par exemple, les principes actifs agissant sur le système nerveux central, les principes actils agissant sur le système cardiovasculaire, les antibiotiques, les anti-viraux et les analgésiques et les anti-inflammatoires.

Les principes actifs agissant sur le système nerveux central sont de préférence choisis parmi les anti-épileptiques, les anti-parkinsonniens, les psycho-stimulants, les psychotropes, les antidépresseurs, les anxiolytiques et les anti-psychotiques par exemple. En particulier, les comprimés sont particulièrement adaptés à des principes actifs tels que la risperidone, le donepezil, la physiostigmine, la rivastigmine, la buspirone, le diazepam, la fluoxetine, le minalcipran, la paroxetine, la sertraline, la venlafaxine, la lamotrigine et la tiagabine.

Les principes actifs agissant sur le système cardiovasculaire sont de préférence choisis parmi les anti-hypertenseurs, les anti-thrombotiques, les anti-agrégants et les hypocholestérolémiants. En particulier les comprimés sont particulièrement adaptes à des principes actifs tels que la fluvastatine, la lovastatine, la pravastatine, la simvastatine, le bezafibrate, le ramipril, le losartan, l'atenolol, le carvedilol, le metoprolol, la nifédipine et le furosémide.

Les analgésiques peuvent être choisis parmi l'hydrocodone, l'hydromorphone, la morphine, l'oxycodone, l'oxymorphone, le tramadol et la gabapentine.

De façon avantageuse, le ou les principes actifs sont intégrés dans la couche active en association avec un agent liant pharmaceutiquement acceptable, tel que ceux habituellement utilisés dans l'industrie pharmaceutique pour la fixation de principes actifs à la surface de supports neutres. Ainsi, la méthode de fixation de la couche active décrite dans le brevet EP 1 200 071 peut tout à fait être employée pour la fixation de la couche active dans le cadre de la présente invention.

De façon préférée, la couche active des microgranules conformes à l'invention est appliquée par pulvérisation d'une solution de principe actif dans un solvant (appelée solution de montage), c'est à dire d'une dispersion tout à fait homogène à l'échelle moléculaire du principe actif Avantageusement, cette solution contient également l'agent liant.

Parmi les agents liants pharmaceutiquement acceptables, on utilisera préférentiellement des agents liants de nature hydrophile et notamment des dérivés de la cellulose tels que l'HPMC, en particulier les grades Pharmacoat^{®} 603 et Pharmacoat^{®} 606, des dérivés de la polyvinylpyrrolidone, en particulier le grade PVP K 30 et également des dérivés du polyéthylene glycol. En particulier, on utilisera comme agent liant le polyéthylene glycol dont le poids moléculaire se situe entre 3000 et 7000, tel que le PEG4000 et le PEG6000 notamment.

Le solvant de la solution de montage pulvérisée doit être adapté au principe actif ou au mélange de principes actifs employés. Ainsi, on pourra par exemple utiliser l'eau, l'éthanol ou encore des solutions hydro-alcooliques de diverses concentrations pour la réalisation de la solution à la base de la couche active. Dans la mesure du possible, on cherchera à adapter le solvant à la nature et aux propriétés physico-chimiques de l'actif afin de limiter le volume de solution nécessaire à la dissolution de l'actif. Cependant, dans la mesure où la présente invention est applicable à des comprimés faiblement, voire très faiblement dosés, la contrainte imposée par des volumes de dissolution trop importants reste marginale.

Dans la mesure du possible, il est préférable d'utiliser des solvants non toxiques et facilement éliminables par évaporation lors du séchage afin qu'il n'en subsiste aucune trace dans les comprimés.

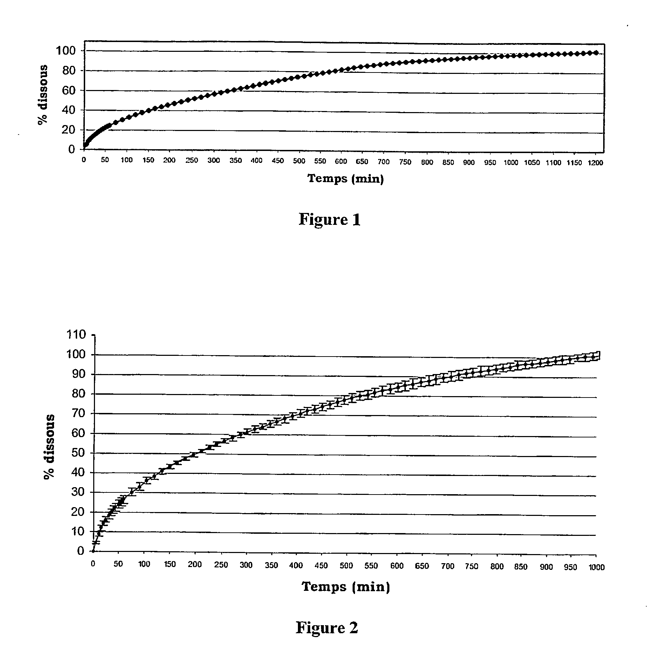

La structure particulière des microgranules selon l'invention et l'étape de compression directe appliquée à ces microgranules permettent l'obtention d'un profil de libération modifiée pour le principe actif, profil directement lié à la nature du polymère employé (Voir figures 1, 2, 3 et 4).

Le phénomène de libération modifiée observé par la demanderesse suivant la nature et les qualités du polymère employé dépend de l'étape de compression, mais est également dépendant du type et de la quantité de polymère employé.

La présente invention permet ainsi d'adapter facilement tant la nature que la quantité de polymère employée pour l'obtention du profil de libération recherché. En effet, que le polymère d'intérêt soit un polymère ralentissant la libération de l'actif tel que la gomme xanthane par exemple, ou qu'il soit un polymère désintégrant comme la crospovidone, le profil de libération de l'actif va être similaire à celui observé pour des comprimés matriciels classiques, obtenus à partir de ces différents polymères,

### Les agents de pelliculage

On peut tout à fait prévoir, compte tenu de leurs caractéristiques physiques similaires à celles de comprimés classiques, de recouvrir les comprimés conformes à l'invention d'une ou plusieurs couches supplémentaires d'un ou plusieurs agents de pelliculage, selon la façon dont on souhaite modifier le profil de libération de l'actif.

Si on envisage de conférer aux comprimés selon l'invention un profil de libération gastrorésistant, on peut tout à fait prévoir de les pelliculer de manière classique par une ou plusieurs couches selon les besoins d'un ou plusieurs agents de pelliculage gastro-résistants.

On pourra utiliser pour ce type de pelliculage tous les excipients classiquement connus de l'homme de l'art pour ces qualités, tels que certains polymères dérivés de l'acide méthacrylique et en particulier les copolymères de l'acide méthacrylique vendus sous le nom commercial Eudragit^{®} L dont le nom chimique est poly(méthacrylique acide, éthylacrylate) ou encore certains dérivés de la cellulose comme l'Hydroxypropylméthyl cellulose phtalate par exemple, ou encore certains dérivés du polyvinyl acétate comme le polyvinyl acétate phtalate. On peut également conférer aux comprimés selon l'invention une libération colonique en utilisant certains polymères dérivés de l'acide méthacrylique et en particulier les copolymères de l'acide méthacrylique vendus sous le nom commercial Eudragit^{®} S et Eudragi^{®} FS dont le nom chimique est poly(méthacrylique acide, méthyl méthacrylate).

### Le procédé de préparation des comprimés

La présente invention a en outre pour objet le procédé de préparation des comprimés précédemment décrits qui comprend les étapes suivantes :
- le support neutre est préalablement humidifié à l'aide d'une solution de mouillage contenant éventuellement un agent liant;
- le polymère est ensuite appliqué à la surface du support neutre par poudrage ;
- la couche polymérique ainsi obtenue set éventuellement séchée pour former un excipient fonctionalisé ;
- une solution de montage comprenant le principe actif et éventuellement un agent liant, est pulvérisée sur la surface de la couche polymérique;
- les microgranules ainsi obtenues sont ensuite séchées, puis directement comprimés ;
- le comprimé ainsi obtenu est éventuellement enrobé d'une ou plusieurs couches d'agent de pelliculage.

En outre, l'étape de compression peut s'effectuer à l'aide de moins de 5% en masse de lubrifiant par rapport à la masse totale des comprimés.

### • Procède de montage de la couche polymérique par poudrage en turbine conventionnelle

La mise en solution de polymères et notamment de polymères hydrophiles gélifiants, tels que les polysaccharides de haut poids moléculaire fait apparaître inévitablement le problème de la viscosité des solutions de montage ; seules de faibles quantités de polymère peuvent être dissoutes puis montées en lit d'air fluidisé sauf à obtenir des volumes de solution de montage incompatibles avec une exploitation industrielle du procède. La concentration en polymère est donc un facteur limitant pour certains polymères si on envisage un montage de la couche polymérique par pulvérisation.

Ainsi, pour la réalisation de la présente invention, l'étape de fixation de la couche polymérique est préférentiellement effectuée par poudrage, ce qui permet d'accroître la quantité de polymère à fixer sur les neutres en s'affranchissant des problèmes de viscosité rencontrés avec de nombreux polymères.

On peut en outre prévoir d'associer au polymère de la couche polymérique une cire ou un de ses dérivés, ou un dérivés d'acide gras du glycérol, ou un mélange de ceux-ci.

Ce procédé consisté à réaliser le montage du polymère sous forme sèche pulvérulente sur les neutres dans une turbine conventionnelle, à l'aide d'une solution de mouillage. La couche active contenant le principe actif avec un agent liant éventuel peut ensuite, de façon classique, être montée par pulvérisation en lit d'air fluidisé ou à l'aide d'autres appareils de montage conventionnels.

La turbine conventionnelle utilisée pour le montage de la couche polymerique est par exemple un récipient de forme sphérique ouvert sur le quart de sa surface et incliné par rapport à l'horizontale. Le montage du polymère s'effectue Manuellement, éventuellement à l'aide d'un liant, dissous dans un solvant. D'une manière préférée, on utilisera un solvant faiblement ou non toxique, tel que l'eau, l'éthanol ou une solution hydro-alcoolique. Cette solution dite "de mouillage" est utilisée pour humidifier les noyaux neutres et favoriser la fixation de la poudre de polymère leur surface.

Le cycle de montage est par exemple le suivant :
- On procède tout d'abord au mouillage de la masse des neutres en rotation dans la turbine par application d'une fine couche de solution de mouillage sur la surface des neutres.
- Puis on passe à l'étape de poudrage de la masse de neutres par dispersion manuelle, d'une certaine quantité de polymères d'intérêt à la surface des neutres. Le polymère se fixe ainsi à la surface du support neutre sous la forme de grains de poudre.
- L'opération de poudrage est renouvelée ou non autant de fois qu'il est nécessaire pour obtenir un "excipient fonctionnalisé" de la teneur voulue en polymère. On procède ainsi à un nombre variable de cycles de mouillage/poudrage qui dépend de la quantité de polymère d'intérêt choisi.

L'avantage du montage en turbine conventionnelle réside dans le fait que le polymère est projetée sous forme sèche et donc qu'aucun phénomène de gélification n'a lieu. On s'affranchit ainsi des problèmes techniques tels que le colmatage des buses de pulvérisation ou la prise en masse des grains neutres, observés lors de la pulvérisation de polymères visqueux.

Pour une répartition homogène du polymère à la surface des neutres, il est préférable que le rapport de taille neutres / grains de polymère ne soit pas trop faible, c'est pourquoi on utilisera préférentiellement des neutres de taille supérieure à 50 µm.

Enfin, une étape de séchage permet d'éliminer le solvant utilisé lors de l'étape de mouillage des neutres. Cette opération s'effectue préférentiellement en externe, c'est à dire qu'une soufflerie d'air à température contrôlée chauffe la paroi de la turbine qui transmet par conduction la chaleur aux neutres, eux-mêmes en agitation.

Le séchage peut également être réalisé en lit d'air fluidisé ou en étuve ou selon tout autre moyen de séchage couramment employé pour le séchage de microgranules. L'opération de séchage peut durer plusieurs heures jusqu'au séchage complet des microgranules montés. La température dans la turbine est maintenue constante, généralement inférieure à 80°C, pour une température au sein de la masse de neutres idéalement proche de 40 °C.

Ainsi, à l'issue de ces étapes, on obtient un "excipient fonctionnalisé" stabilisé, c'est à dire un support neutre sur lequel est fixé de façon stable par l'intermédiaire d'un liant, le polymère d'intérêt choisi.

La figure 6 est une photographie prise en microscopie électronique de microgranules conformes l'invention après l'étape de poudrage du polymère.

Cela revêt une importance toute particulière en terme de fabrication industrielle, puisqu'on peut tout a fait envisager de fabriquer ces excipients de façon indépendante, de les stocker et/ou de les transporter jusqu'à l'endroit où ils devront recevoir la couche active conforme à la présente invention avant d'être comprimés. Les excipients fonctionnalisés de la présente invention présentent donc une facilité d'emploi non négligeable en terme industriel.

### • Préparation de la solution de montage

L'étape dite de montage de la couche active conformément à la présente invention permet d'obtenir des microgranules faiblement doses et dont la teneur en actif est à la fois précise et uniforme. On s'affranchit en particulier des problèmes de démélange avant l'étape de compression directe en appliquant sur des noyaux neutres préalablement recouverts d'une couche polymérique, le principe actif sous forme dissoute dans la solution de montage, ce qui permet de figer le mélange sans granulation.

La solution dite de montage est la solution dans laquelle le ou les principes actifs vont être dissous ou mis en suspension (dispersés) et qui va être pulvérisée à la surface des microgranules "montés", encore appelés "excipients fonctionnalisés" dans la présente demande. Cette solution contient avantageusement un agent liant conventionnel dissout également.

Si le principe actif est dissout, il faut prendre en compte la solubilité de l'actif lors de la préparation de la solution de montage. En effet, il faut s'assurer que l'actif est totalement dissout avant de procéder sa pulvérisation. Ainsi la quantité de solution de montage est avantageusement choisie comme étant égale à au moins 1,5 fois la quantité nécessaire pour atteindre la concentration de saturation en actif.

### • Montage de la couche active

Le principle actif est appliqué sur les granules "poudrés" (c'est à dire recouverts du polymère d'intérêt) de façon conventionnelle par pulvérisation, en lit d'air fluidisé par exemple. D'une manière générale, ce procédé repose sur la pulvérisation simultanée au travers d'une buse, du ou des principes actifs et éventuellement d'un liant qui sont parfaitement dissous ou dispersés dans la solution de montage, ce qui garantit pour cette étape du procédé une parfaite homogénéité de teneur.

Le temps nécessaire au montage est très variable et dépend de la quantité d'actif à pulvériser et de sa solubilisé dans la solution de montage. De manière générale il est compris entre 1 et 10 heures.

A l'issue de l'étape de montage, les microgranules sont séchés en lit d'air fluidisé puis tamisés avant l'étape de compression directe.

Ainsi, le temps de réalisation du procédé est fonction à la fois de la quantité de polymère à appliquer, mais également de la quantité d'actif à pulvériser et de la solubilité de l'actif dans le solvant de montage. C'est pourquoi il est utile d'adapter la solution de montage aux conditions de solubilité de l'actif, afin de diminuer les volumes de solution à pulvériser et donc le temps nécessaire à l'étape de montage. Cc procédé peut généralement se décomposer de la manière suivante :
- phrase 1 : Poudrage : entre 1 et 3 heures pour la constitution de la couche polymérique.
- phase 2 : Séchage (élimination du solvant de montage) : entre 0,5 et 12 heures suivant la quantité de solvant utilisée.
- phase 3 : Montage : entre 1 est 10 heures pour la pulvérisation de la solution de principe actif
- Phase 4 Séchage entre 15 minutes est 12 heures suivant la quantité de solvant utilisée.
- Phase 5 : compression directe des microgranules ainsi obtenus.

Comme cela a été mentionné précédemment, du fait de la stabilisation de l'excipient fonctionnalisé à la suite de l'étape de poudrage, il est tout à fait envisageable de réaliser les étapes de montage et de compression de façon indépendante des étapes antérieures (phase 1 et 2). Cette caractéristique permet d'envisager le transport et la manutention aisée des excipients fonctionnalisés obtenus à l'issue des deux première études du procédé.

### • Compression

Avant l'étape de compression, les microgranules montés sont préférentiellement lubrifiés à l'aide d'un lubrifiant conventionnel, tel que le stéarate de magnésium par exemple. Le lubrifiant est utilisé en général en très faible quantité, et d'une manière générale à moins de 5 % en masse de la masse fïnale du comprimé. Les microgranules et le lubrifiant sont par exemple mélangés de façon conventionnelle dans un mélangeur rotatif.

La compression proprement dite peut être effectuée sur une presse alternative ou sur une presse rotative. Les forces de compression appliquées sont préférentiellement comprises entre 50 et 500 Mpa (Méga Pascal). L'étape de compression n'est pas limitante : on peut ainsi appliquer de fortes contraintes de compression sans risque d'altérer la structure du comprimé comme c'est le cas dans les systèmes "réservoir". En effet, non seulement les microgranules neutres ont un excellent comportement en compression, comme cela est connu depuis le brevet EP1 200 0711, mais encore la configuration des microgranules conformes à l'invention est-elle particulièrement adaptée à d'importantes forces de compression.

Bien entendu, les contraintes de compression doivent cependant être adaptées à la dureté et à la cohésion recherchées pour les comprimés selon l'invention.

### Les tests de dissolution et de dosage

D'une manière générale, les conditions de dosage et de dissolution des comprimés conformes à l'invention sont celles prescrites par les différentes pharmacopées, européenne, américaine ou japonaise.

Ainsi, pour déterminer les cinétiques :de libération des différents systèmes étudiés, un appareil de dissolution conventionnel à palettes ou à paniers peut être employé. Cet appareil peut être relié à un spectrophotometre, capable d'effectuer automatiquement le prélévement et la mesure de l'absorbance en UV des solutions testées. Cette mesure automatique n'est possible que lorsque la quantité de principe actif est suffisamment importante et lorsque le principe actif n'absorbe pas en UV à la même longueur d'onde qu'un des excipients de la solution. Cette mesure automatique de l'absorbance en UV permet de déterminer par rapport à une solution de référence de concentration connue, la quantité d'actif libéré dans le milieu de dissolution.

De tels prélèvements automatiques du milieu de dissolution au cours de la cinétique peuvent être effectués de la manière suivante :
- un prélèvement toutes les 5 minutes durant la première heure
- un prélèvement toutes les 15 minutes jusqu'à l'apparition d'un plateau pour 100% d'actif dissous

Lorsque les conditions ne s'y prêtent pas, le dosage du principe actif libéré dans le milieu de dissolution est effectué par prélèvements manuels analysés ensuite par HPLC (High Performance Liquid Chromatography).

### Les tests de désagrégation

Dans le cas de comprimés fabriqués à partir de polymères désintégrants, on utilisera un test classique de désagrégation tel que préconisé par la pharmacopée européenne pour juger de la vitesse de désintégration des comprimés conformes à l'invention.

Dans un tel test, les comprimés sont placés dans des tubes cylindriques creux au fond desquels une grille métallique comportant des mailles de 2 mm retient le comprimé inséré dans chacun des tubes. L'ensemble de l'appareillage est plongé dans un bain aqueux. Les tubes sont alors soumis à un mouvement vertical alternatif régulier d'environ 30 cycles par minute.

On mesure le temps de désagrégation totale du comprimé lorsqu'il ne reste plus à la surface de la grille aucun résidu de comprimé.

Les comprimés selon l'invention sont adaptés à l'administration par voie orale de médicaments, notamment à l'administration par la voie sublinguale ou transmucosale, lorsque par exemple la couche polymérique contient des polymères muco-adhésifs. En particulier, les comprimés conformes à la présente invention sont particulièrement utiles pour l'administration de principes actifs faiblement dosés, comme les hormones et leurs dérives qui doivent parfois être administrées à des doses de l'ordre du microgramme, mais également les vitamines et certains médicaments du système nerveux central ou du système cardiovasculaire notamment.

La figure 1 représente le profil de libération du kétoprofène, utilisé comme principe actif modèle, obtenu à partir de comprimés conformes à l'invention obtenues selon le procédé de l'exemple 1, dont la couche polymérique comprend 5% en masse de gomme xanthane (grade Rhodigel^{®} 200) par rapport à la masse des supports neutres.

La figure 2 représente le profil de libération du kétoprofène, utilisé comme principe actif modèle, obtenu à partir des comprimés conformes à l'invention obtenus selon le procédé de l'exemple 2, dont la couche polymérique comprend 6% en masse de gomme xanthane (grade Rhodigel^{®} 200) par rapport à la masse des supports neutres,

La figure 3 représente le profil de libération du kétoprofène, utilisé comme principe actif modèle, obtenu à partir des comprimés conformes à l'invention obtenus selon le procédé de l'exemple 4, dont la couche polymérique comprend 5% en masse d'HPMC de viscosité 100000 mPa s dans une solution aqueuse à 2 % m/m à 20°C (grade Metolose^{®} 90 SH) par rapport à la masse des supports neutres.

La figure 4 représente le profil de libération du kétoprofèno obtenu à partir de microgranules de kétoprofène « en vrac », c'est-à-dire non comprimés, comprenant 2% en masse de gomme xanthane (grade Rhodigel^{®} 200) par rapport à la masse des supports neutres. Ces microgranules sont obtenus conformément au procédé décrit dans l'exemple 1, mais sans l'étape de compression d).

La figure 5 est la représentation schématique de la formation du réseau polymérique des comprimés selon la présente invention. Plus particulièrement, la figure 5 représente la formation de percolation au sein des comprimés lors de l'argumentation de la quantité du polymère (note ; les effets de la compression des neutres ne sont pas pris en compte).

La figure 6 est la photographie prise en microscopie électronique d'excipients fonctionalisés conformes à l'invention après l'étape de poudrage du polymère.

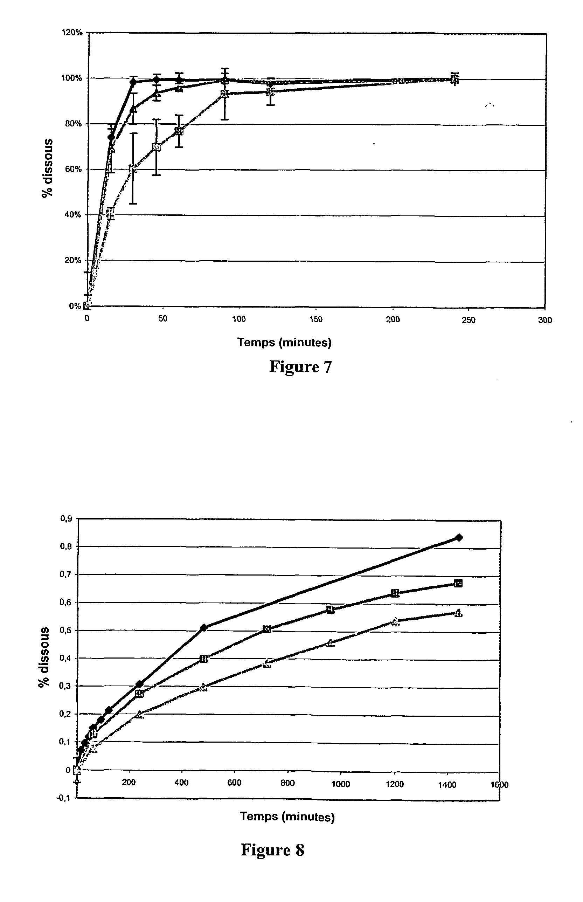

La figure 7 représente le profil de libération du ketoprofène obtenu à partir des comprimés conformes à l'invention et obtenus selon le procédé de l'exemple 5 dont la couche polymérique comprend respectivement 5%, 10% et 15% en masse d'HPMC par rapport à la masse des supports neutres.
---◆--- Lot B7 (5% d'HPMC)-250MPa
---▲--- Lot B8 (10%d'HPMC)-250MPa
---■--- Lot B9 (15% d'HPMC)-250MPa

La figure 8 représente le profil de libération du kétoprofène obtenu à partir des comprimés conformes à l'invention et obtenues selon le procédé de l'exemple 6, dont la couche polymérique comprend respectivement 2%, 5% et 10% de Carbopol® 971 P par rapport à la masse des supports neutres. Ces cinétiques sont obtenues pair prélèvement du milieu de dissolution et dosage des prélèvements par HPLC (chromatographie liquide haute performance) puis détection UV.
---◆--- Lot C1-2% de Carbopol
---▲--- Lot C2-5% de Carbopol
---■--- Lot C3-10% de Carbopol

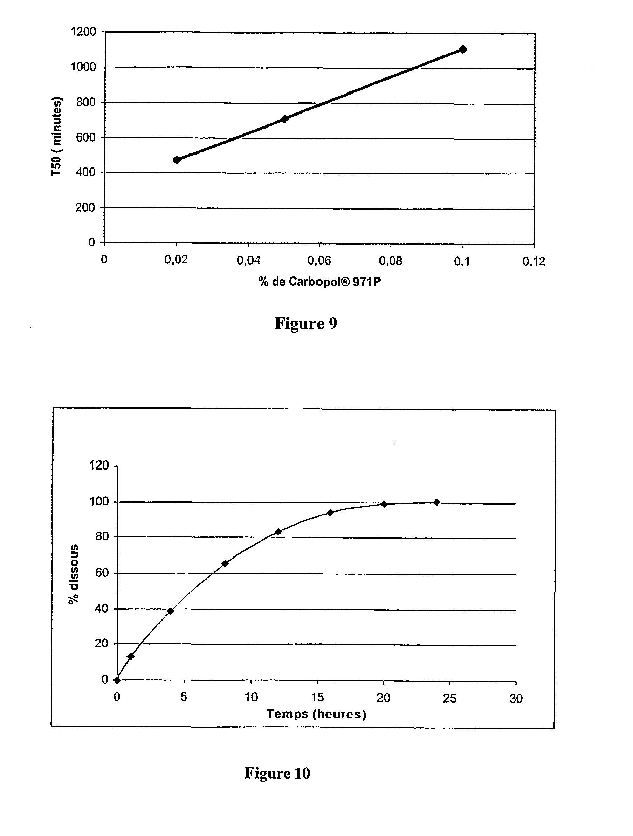

La figure 9 représente la variation du T50 en fonction du pourcentage de Carbopol® 971 P pour les comprimés selon l'exemple 6.

La figure 10 représente le profil de libération du principe actif 23015, obtenus à partir des comprimés conformes à l'invention obtenus selon le procédé de l'exemple 3, dont la couche polymérique comprend 15 % en masse de gomme xanthane (grade Rhodigel^{®} 200) par rapport à la masse des supports neutres.

### Exemples

### A) Préparation de comprimés

### Exemple 1 : Comprimés de microgranules de kétoprofène à libération prolongée à base de gomme xanthane 5 % (Lot B4)

### a) Montage de la gomme xanthane par poudrage

Deux kilogrammes de noyaux neutres de taille compris entre 400 et 500 µm (Suglets^{®} fournis par la société NP Pharm, France) sur lesquels va être montée la couche polymérique sont introduits dans une turbine conventionnelle et mis en rotation.

Le polymère utilisé ici est un polymère hydrophile à propriétés gélifiantes : la gomme xanthane (fournie par la société Rhodia, France vendue sous le grade Rhodigel^{®} 200) dont 92 % des particules ont une taille inférieure à 75 µm.

Les noyaux neutres sont tout d'abord mouillés à l'aide de 300 grammes d'une solution alcoolique de polyvinylpyrrolidone à 15 % (grade PVP K 30, fournie par la société ICI, France), soit 45 grammes de PVP K 30.

Cette solution liante est appliquée par pulvérisation à la surface des neutres en rotation par l'intermédiaire d'un pulvérisateur manuel.

5 % en masse de la masse de microgranules neutres, soit 100 grammes de gomme xanthane sous forme de poudre sèche sont introduits manuellement dans la turbine en rotation immédiatement après l'étape de mouillage des noyaux neutres.

Ces étapes de mouillage/poudrage s'effectuent en plusieurs cycles répétés comprenant chacun une première phase de pulvérisation d'une partie de la solution de polyvinylpyrrolidone à la surface des noyaux neutres (mouillage), suivie d'une phase de poudrage proprement dite correspondant à la projection d'une partie de la poudre de gomme xanthane.

L'opération de poudrage dure environ 2 heures et comprend autant de cycles de mouillage / poudrage que nécessaire à la fixation de la quantité déterminée de gomme xanthane. La rotation de la turbine est maintenue durant toute la durée de la phase de poudrage.

A l'issue de l'opération de poudrage proprement dite, une étape de séchage des neutres « poudrés » intervient.

Le séchage est réalisé en maintenant à température constante la masse de neutres pendant 8 heures. La température de séchage est idéalement d'environ 40°C au coeur de la masse de neutres.

### b) Montage de la couche active

Les neutres montés avec la couche polymérique de gomme xanthane à 5 % sont ensuite soumis à l'étape de montage du principe actif. Cette étape est réalisée en lit d'air fluidisé (LAF) Kugelcoater^{®} de la société Hüttlin (Allemagne), les paramètres de la pulvérisation sont récapitulés dans le tableau 1.

**Tableau 1: Récapitulatif des paramètres de montage au lit d'air fluidisé Hüttlin pour le lot monté avec 5 % de gomme xanthane.**

| | |
|---|---|
| **Température entrée d'air (°C)** | 50 |
| **Température sortie d'air (°C)** | 37 à 39 |
| **Température produit (°C)** | 38 à 40 |
| **Pression de nébulisation (bar)** | 0,8 |
| **Débit d'air de fluidisation (m³/h)** | 195 |
| **Température de séchage (°C)** | 45 |
| **Débat d'air en séchage (m³/h)** | 195 |
| **Temps de séchage (min)** | 30 |

Le principe actif utilisé est le kétoprofène (2-(3-benzylphényl)propionique) fourni par SIMS (Italie) à une concentration de 0,4 % par rapport à la masse des noyaux neutres, soit 8 grammes pour 2 kg de noyaux neutres.

Le kétoprofène étant quasiment insoluble dans l'eau désionisée (solubilité inférieure à 0,1g/L selon la Pharmacopée Européenne), mais présentant une solubilité pH dépendante on utilise un tampon pH 8 pour permettre sa solubilisation lors de la préparation de la solution de montage.

La composition du milieu tamponné est récapitulée dans le tableau 2.

**Tableau 2: Composition du tampon utilisé pour la dissolution du kétoprofène**

| **Tampon pH 8** | |
|---|---|
| KH₂PO₄ | 6,805g |
| H₂O désionisée | 900 mL |
| NaOH 1N | environ 50 mL |
| (ajustement à l'aide d'un pH-mètre) | |
| H₂O désionisée | qsp 1 L |

Le kétoprofène est dissous dans la solution de montage contenant en outre comme agent liant le polyéthylène glycol de poids moléculaire égal à 6000 (PEG₆₀₀₀, ICI, France) également sous forme dissoute.

Le liant est utilisé à raison de 5 % en masse par rapport à la masse de neutres engagés, soit 100 grammes.

La solution de montage est donc constituée de 2000 grammes de tampon pH 8 dans lesquels sont dissous 8 grammes de kétoprofène et 100 grammes de PEG₆₀₀₀. Cette solution est pulvérisée à la surface des microgranules suivant les paramètres récapitulés dans le tableau 1.

### c) Séchage ct tamisage

Une fois montés avec le principe actif, les microgranules sont sèchés pour éliminer toute trace de solvant de montage résiduel. La température de séchage est de 45 °C, appliquée de façon continue pendant 30 minutes.

A l'issue de l'étape de séchage, les microgranules sont tamisés sur une grille de 0,625 mm, afin de garantir une homogénéité de taille parfaite au sein du lot.

### d) Compression

Avant compression, les neutres montés sont lubrifiés avec du stéarate de magnésium à raison de 0,125% m/m. Le mélange est réalisé dans un mélangeur de type Turbula^{®} pendant 2 min à 48 tr/min.

La compression proprement dite est effectuée sur les microgranules lubrifiés à l'aide d'une presse alternative de type Frogerais OA instrumentée et montée avec des poinçons plats de 1 cm² de surface. La hauteur de remplissage de la matrice est réglée pour obtenir des comprimés d'une masse d'environ 500 mg. L'instrumentation et le logiciel associé (Pecamec^{®}, version 4.3., 2001, J2P Instrumentation) permettent de faire, en continu, un relevé des forces appliquées par les poinçons supérieur et inférieur, des forces résiduelles et d'éjection, ainsi qu'un relevé des déplacements des poinçons. La contrainte de pression appliquée de 250 MPa permet d'avoir une cohésion des comprimés suffisante.

### e) Dosage et dissolution des comprimés

Pour déterminer les cinétiques de libération des différents systèmes étudiés, un appareil de dissolution (Dissolutest^{®}, Sotax, Suisse) en paniers tournants, équipé de 7 cuves dont les milieux de dissolution (500 mL) sont thermostatés à 37°C a été employé. Cet appareil relié à un spectrophotomètre UVIKON 922 (Italie) est capable d'effectuer automatiquement le prélèvement et la mesure de l'absorbance en UV des solutions contenues dans les différents bols. L'un des bols contient une solution de concentration connue en actif et dont l'absorbance mesurée tout au long de l'expérience constitue la référence à partir de laquelle est calculée le pourcentage d'actif dissous dans le bol contenant la solution étudiée.

Le milieu de dissolution choisi est un tampon de pH 6,8. Les prélèvements automatiques du milieu au cours de la cinétique sont effectués de la manière suivante :
- un prélèvement toutes les 5 minutes durant la première heure
- un prélèvement toutes les 15 minutes jusqu'à l'apparition d'un plateau pour 100 % d'actif dissous.

De manière à accroître la sensibilité du dosage et étant donné que les comprimés sont faiblement dosés, chaque bol de dissolution contient 3 comprimés. En outre, la cinétique de dissolution est effectuée sur 3 bols. Le tableau 3 regroupe l'ensemble des paramètres utilisés pour réaliser le dosage et les cinétiques du système étudié.

Les résultats de ces mesures sont traduits sur la Figure 1.

On constante que la libération du principe actif est très prolongée, puisqu'il n'est libéré à 100 % qu'au bout de 16 heures environ.

La composition qualitative et quantitative des différents excipients des comprimés obtenus selon l'exemple 1 sont récapitulés dans le tableau 6.

Les caractéristiques de ces comprimés sont récapitulées dans le tableau 7.

**Tableau 3: méthodes analytiques et conditions opératoires utilisées pour caractériser les microgranules montés (vrac) et les comprimés à base de kétoprofène,**

| | **Dosage** (pour uniformité de teneur) | | **Dissolution** | |
|---|---|---|---|---|
| | *Vrac* | *Comprimés* | *Vrac* | *Comprimés* |
| **Nombre d'unités analysées** | 3 | 3 | 3 | 3×3 |
| **Méthodes** | Détection UV à 257 mn *(1)* dissolution dans un mélange méthanol / eau (3/1) | | UV continu à 260 nm *(2)* 500 ml pH 6,8 100 rpm / pales 37°C | UV continu à 260 nm *(2)* 500 ml pH 6,8 50 rpm/paniers 37°C |
| **Appareillage** | Spectrophotomètre UVIKON 922 (Italie) | | Dissolutest SOTAX (Suisse) Spectrophotomètre UVIKON 922 (Italie) | |

| | | | | |
|---|---|---|---|---|
| *(1)* Le choix de la longueur d'onde s'est fait après établissement du spectre du Kétoprofène dans un mélange méthanol/eau (3/1). *(2)* Le choix de la longueur d'onde s'est fait après établissement du spectre du Kétoprofène dans le milieu de dissolution à pH 6,8. | | | | |

### Exemple 2: Comprimés de kétoprofène à base de gomme xanthane 6% (lot D5)

Dans cet exemple, un travaille exactement comme dans l'exemple 1, mis à part que la quantité de gomme xanthane utilisée pour la constitution de la couche polymérique des microgranules vaut 6 % en masse par rapport à la masse des supports neutres engagés.

La composition qualitative et quantitative des différents excipients des comprimés obtenus selon l'exemple 2 sont récapitulés dans le tableau 6.

Les caractéristiques de ces comprimés sont récapitulées dans le tableau 7.

Les cinétiques de libération du kétoprofène sont déterminées dans les mêmes conditions que celles de l'exemple 1, Les résultats de ces mesures sont traduits sur la figure 2.

### Exemple 3 : Comprimés de principe actif (ref_; 23015) à base de gomme xanthane 15 % (lot B 10)

Dans cet exemple, on travaille exactement comme dans l'exemple 1, mis à part que la quantité de gomme xanthane utilisée pour la constitution de la couche polymérique des microgranules vaut 15 % en masse par rapport à la masse des supports neutres engagés et que le principe engagé n'est plus le kétoprofène, mais un principe actif référencé sous le numéro 23015.

La composition qualitative et quantitative des différents excipients des comprimés obtenus après compression selon l'exemple 3 sont récapitulés dans le tableau 4 suivant :

**Tableau 4: Composition qualitative et quantitative des comprimés de microgranules à base de principe actif 23015 obtenus avec 15 % de gomme xanthane en masse par rapport à la masse des supports neutres engagés, soit 11, 65 % de la masse totale de microgranules. (lot B10)**

| **Type** | **Nom chimique** | **Nom commercial** | **Quantité (g) (matière sèche)** |
|---|---|---|---|
| Support | Sphère de sucre | Neutre 400-500 | 77,664 |
| Principe actif | 23015 | N/A | 1,230 |
| Polymère matriciel | Gomme xanthane | Rhodigel 200 | 11,651 |
| Liant | Povidone | PVPK30 | 3,160 |
| | PEG 6000 | Renex PEG 6000 | 6,160 |
| Tensioactif | Polysorbate 80 | Polysorbate 80 | 0,010 |
| Lubrifiant | Stéarate de magnésium | N/C | 0,125 |
| TOTAL | | | 100,000 |

### Dissolution

Les comprimés ainsi obtenus ont ensuite été soumis à un test de dissolution dans 900 ml de milieu à pH 6.8 (dans une fiole de 1 L, 6.805 g de KH2PO4, 22,4 ml de NaOH IN et 900 ml d'eau osmosée ; Eventuellement ajusté à pH 6.8 avec de la sourde puis complété à 1 L avec l'eau),

Chaque comprimé est placé dans un panier, tournant à 50 rpm.

Puis, à 1, 4, 8, 12, 16_{,} 20 et 24 heures, le milieu de dissolution est prélevé et le prélèvement est dosé par HPLC (Chromatographie Liquide Haute Performance) avec détection UV.

Les résultats obtenus sur 3 vase de dissolution sont récapitulés dans le tableau 5 suivaut :

**Tableau 5 : Profil de dissolution des comprimés de microgranules de principe actif 23015 poudrés avec 15 % de gomme xanthane.**

| **temps [h]** | 0 | 1 | 4 | 8 | 12 | 16 | 20 | 24 |
|---|---|---|---|---|---|---|---|---|
| **Moyenne [%]** | 0 | 13.7 | 38,7 | 65.3 | 83.5 | 94.1 | 98.9 | 100,6 |
| **Coefficient Variation [%]** | *0* | *5.2* | *0.9* | *1.3* | *0.5* | *0.7* | *1.3* | *0.9* |

Dans cet exemple, les comprimés de microgranules conformes à l'invention, présentent un T50 égal à 340 min soit 5h40min et un T80 égal à 675 min soit 11h15 min.

Ces résultats sont représentes sur la figure 10.

### Exemple 4; Comprimé de microgranules de kétoprofène à base de 5% d'HPMC 100 000 mPa.s (Lot B6)

### a) Montage de l'HPMMC poudrage

Comme dans l'exemple 1, une masse de 2 kg de neutres de 400 à 500 µm (Suglets^{®}) est mise en rotation dans une turbine conventionnelle.

Puis une étape de mouillage similaire à celle décrite dans l'exemple 1 est réalisée à partir d'une solution aqueuse liante de PVP K30 à 15 % (169 grammes de solution liante dont 25 grammes de PVP).

Le polymère d'intérêt est un polymère hydrophile à propriétés gélifiantes : l'HPMC vendue sous le nom Metolose^{®} 90 SH, dont la viscosité vaut 100000 mPa.s.(milli Pascal secondes) pour une solution aqueuse à 2% m/m à 20°C.

100 grammes d'HPMC (soit 5 % en masse par rapport à la masse des neutres engagés) sont introduits manuellement dans la turbine en rotation. Comme décrit dans l'exemple 1, une phase de séchage termine la séquence de poudrage.

### b) Montage du kétoprofène par pulvérisation

Le principe actif utilisé est le ketoprofène à raison de 0,4 % de la masse de neutres engagés, soit 8 grammes. La couche active est montée en lit d'air fluidisé (LAF) comme dans l'exemple 1, à partir d'une solution contenant à l'état dissout le principe actif et l'agent liant (PEG₆₀₀₀ à 5 % en masse par rapport à la masse des neutres) dans le tampon pH 8 (2000 grammes).

### c) Séchage et tamisage

Une phase de séchage est une phase de tamisage identiques à celles de l'exemple 1 sont réalisées sur la masse de microgranules avant la compression.

### d) Compression

Avant compression, les neutres montés sont lubrifiés avec du stéarate de magnésium (stm) ; 0,125% m/m. Le mélange est réalisé dans un mélangeur de type Turbula^{®} pendant 2 min à 48 tr/min. Le mélange est ensuite comprimé sur une presse alternative de type Frogerais OA en appliquant une force de compression de 250 M Pa.

### c) Dosage et dissolution des comprimés

Comme dans l'exemple 1, les comprimés obtenus sont dissous dans un Dissolutest^{®} et la quantité de principe actif libéré dans le milieu est mesurée en fonction du temps.

Les résultats de ces mesures sont traduits sur la figure 3.

On constate que le profil de libération du principe actif est également de type prolongé, puisque seulement 80 % du principe actif est libéré au bout de 30 minutes comme cela est représenté sur la figure 3.

### B) Composition qualitative et quantitative des différents excipients des comprimés obtenus selon l'exemple 4, et leurs caractéristiques de dissolution.

**Tableau 6: Composition d'un lot à base d'hydroxypropylméthylecellulose (tous les pourcentages sont des rapports de la masse du produit par rapport à la masse des neutres engagés soit 2000 g)**

| | | **Phase 1 : Poudrage** | | | **Phase 2 : LAF** | | |
|---|---|---|---|---|---|---|---|
| **Exemple** | **Numéro du lot** | **Quantité de polymère** | **Quantité de solution liante** | **Quantité de PVP (15 % de la sol.)** | **Quantité d'actif** | **Quantité de PEG** | **Quantité de solvant** |
| **4** | B6 | 5 % HPMC soit 100 g | 169 (g) | 25 (g) | 0,4 % | 5 % | 2000 (g) |

**Tableau 7 : Mesures de certaines caractéristiques des comprimés obtenus selon l'exemple 4.**

| | **B6** |
|---|---|
| **Rendement massique global (%)** | 95,8 |
| **Masse moyenne des comprimés (mg) (n=20)** | 506,3 -/- 7,1 |
| **Titre théorique de départ (mg / cp)** | 1,77 |
| **Titre réel obtenu (mg/cp/) (n= 3)** | 1,74 +/- 0,02 |
| **T₅₀ (minutes) (*)** | 14 |
| **T 80 (minutes) (*)** | 30 |

| | |
|---|---|
| (*) le T₅₀ et le T₈₀ correspondent respectivement au temps au bout duquel 50 et 80 % du principle actif sont dissous dans le milieu de dissolution. | |

### Exemple 5 : Comparaison des profils de dissolution de comprimés de microgranules de kétoprofène à base de 5%, 10 % et 15% d'HPMC 100 000 mPa.s (Lots B7, B8, et B9)

Dans cet exemple, la demanderesse a comparé les cinétiques de dissolution in vitro de comprimés de microgranulcs de kétoprofène obtenus selon la méthode décrite dans l'exemple 4 mais à partir de noyaux neutres de départ d'un diamètre compris entre 250 et 355 µm, et poudrés avec respectivement 5%, 10% et 15% d'HPMC Métolose® 90SH (Shin Etsu, Japon, Lot 0203021) dont la viscosité vaut 100000 mPa.S pour une solution aqueuse à 2% m/m à 20°C. Ces comprimés sont obtenus après avoir été soumis à une contrainte de compression de 250 MPa

Comme dans l'exemple 1, les comprimés obtenus sont dissous dans un Dissolutest® et la quantité de principe actif libéré dans le milieu est mesurée en fonction du temps.

### Détermination de l'influence de la concentration en polymère

La figure 7 représente les cinétiques de libération des comprimés issus des lots B7, B8 et B9.

Le tableau 8 compare également les T50 et T80 mesures pour chaque lot.
T₅₀ = temps au bout duquel 50% du principe actif est libéré.
T₈₀ - temps au bout duquel 80% du principe actif est libéré.

La figure 7 montre que les cinétiques de dissolution des lots B7 et B8 sont assez semblables et de type faiblement prolongé, puisque 85 à 95 % du principe actif est libéré en 30 minutes.

Par contre, la cinétique de libération du lot B9, est bien ralentie par rapport aux deux autres lots : le T50 du lot B9 est jusqu'à deux fois plus élevée que le T50 des lots B7 et B8 (Tableau 8).

Ainsi, la demanderesse a mis en évidence que la quantité d'HPMC 100000 mPa.s n'influence vraiment la cinétique de libération qu'à partir d'un certain seul compris entre 10 et 15% m/m de ce polymère. Au-delà de ce seuil, la cinétique de libération du PA est bien ralentie et on observe un véritable profil à libération prolongé. En outre, la demanderesse a remarqué que ce seuil varie en fonction de la taille des noyaux neutres de départ.

En effet ce n'est qu'à partir de cette plage de concentrations que la couche de polymère est assez dense pour former un véritable réseau responsable de la libération de l'actif selon un profil vraiment prolotagé.

**Tableau 8: Estimation des temps de libération de 50% et 80% d'actif pour les 3 lots étudiés.**

| **Lot** | **T50 (minutes)** | **T80 (minutes)** |
|---|---|---|
| **B7** | 10,1 | 24,4 |
| **B8** | 10,9 | 26,5 |
| **B9** | 24,9 | 77.2 |

### Exemple 6 : Comprimés de microgranules de kétoprofène à base de 2%, 5% et 10% de Carbopol® 971 P (lots C1, C2, et C3)

Dans cet exemple, la demanderesse a mesuré les profils de dissolution du kétoprofène à partir de comprimés de microgranules obtenus par poudrage selon la méthode décrite dans l'exemple 5, d'un polymère synthétique de la famille des carbomères : le Carbopol® 971 P (Noveon, USA, Lot 0308024) qui peut être utilisé dans des formulations pour comprimés à libération prolongée.

Les lots C1, C2 et C3 représentent respectivement des comprimés de microgranules poudrés avec 2%, 5 % et 10 % de ce carbomère et comprimés à 150 MPa.

Les neutres montés avec la couche polymérique de Carbopol® 971 P sont ensuite soumis à l'étape de montage du kétoprofène. Comme dans l'exemple 1, cette étape est réalisée en lit d'air fluidisé (LAF) Kugelcoater® de la société Hüttlin (Allemagne).

La Figure 8 représente les cinétiques de libération des comprimés issus des lots C1 à C3 dont la quantité en Carbopol® varie de 2% à 10%. Ces cinétiques sont obtenues par prélèvement du milieu de dissolution et dosage des prélèvements par HPLC (chromatographie liquide haute performance) puis détection UV. Cette figure montre que la cinétique de libération de l'actif est d'autant plus lente que la quantité de Carbopol est importante.

En effet, le T50 du lot C1 est de 8h tandis que le T50 du lot C3 (qui contient cinq fois plus de polymère que le lot C1) a un T50 d'environ 18h (voir tableau 9).

Cet exemple montre que l'utilisation de ce polymère permet d'observer des cinétiques de libération très prolongées : les T80 des trois lots sont tous au-delà de 20 heures et ceci sans qu'il y ait une amorce de plateau.

Par ailleurs, la demanderesse a mis en évidence, comme le montre la figure 9 que pour ce polymère, il existe une variation linéaire du T50 en fonction de la quantité de polymère utilisée.

Cette propriété est particulièrement avantageuse dans la mesure où elle peut être utilisée afin de connaître le T50 d'un pourcentage de Carbopol® donné ou, inversement, d'évaluer quel pourcentage de Carbopol® on doit utiliser pour obtenir un T50 déterminé.

**Tableau 9 : Estimation des temps de libération de 50% et 80% d'actif pour les 3 lots poudrés au Carbopol® 971 P étudiés.**

| **Lot** | **T50 (heures)** | **T80 (heures)** |
|---|---|---|
| **C1** | 8 | 22 |
| **C2** | 12 | Au delà de 24 heures |
| **C3** | 18 | Au-delà de 24 heures |

### Exemple 7 : Comprimés de microgranules à base de 0,5% et 1% d'agents désintégrants (lots D1, D2, et D3)

Dans cet exemple, la demanderesse s'est attachée à démontrer le caractère désintégrant de comprimés de microgranules obtenus conformément à la méthode décrite dans l'exemple 5, mis à part qu'aucun principe actif n'est fixé sur les granules, préalablement poudrés : l'étape de montage par pulvérisation au LAF n'est pas réalisée dans cet exemple.

Le détail de la composition de différents lots est répertorié dans le tableau 10. Le poudrage s'effectue comme décrit dans les exempler précédents, avec 3 types d'excipients à propriétés désintégrantes : la polyvinylpyrrolidone réticulée (lot D1), le carboxyméthylamidon sodique (lot D2) et la croscarmellose sodique (lot D3).

Pour cela, il a été testé, pour chaque type de désintégrant 2 sous-lots composés chacun de comprimés de microgranules poudrés à 0,5% (lots D1, D2 et D3) et 1% (lots D1', D2' et D3') de polymères désintégrant.

Un test de désagrégation a été réalisé dans les conditions préconisées par la Pharmacopée européenne. Dans un tel test, les comprimés sont placés dans des tubes cylindriques creux au fond desquels une grille métallique comportant des mailles de 2 mm retient le comprimé inséré dans chacun des tubes. L'ensemble de l'appareillage est plonge dans un bain aqueux. Les tubes sont alors soumis à un mouvement vertical alternatif régulier d'environ 30 cycles par minute.

On mesure le temps de désagrégation totale du comprimé lorsqu'il ne reste plus à la surface de la grille aucun résidu de comprimé,

Pour chacun de ces lots, a été déterminé le temps de désagrégation du dernier comprimés désagrège sur un total de 6 comprimés par sous-lots.

Par ailleurs, la demanderesse a testé pour chaque sous-lot, 2 valeurs de force de compression différentes exprimées dans le tableau 11 sous la forme du résultat en terme de cohésion acquise, c'est à dire d'une contraintes de rupture d'environ 0,3 MPa et d'une contrainte de rupture d'environ 0,5 MPa.

Lot D1 et D'1: la polyvinylpyrrolidone réticulée utilisée respectivement à 0.5 et 1% pour la constitution du lot D1 est vendue sous le nom Polyplasdone® XL10 (ISP, Suisse, Lot 0404046) qui est un désintégrant insoluble dans l'eau généralement utilisé à des concentrations allant de 2 à 5% dans des comprimés préparés par granulation humide, sèche ou compression directe.

Ce polymère est caractérise par une grande capacité d'hydratation mais avec une faible tendance à former un gel.

Lot D2 et D'2: dans cet exemple, dans un souci de comparaison avec les autres désintégrants testés, la demanderesse a utilisé le carboxyméthylamidon sodique Explosol® (Blanver, Brésil, 0311044) à 0,5 et 1% en m/m pour la constitution des comprimés de microgranules des lots D2 et D'2.

Les carboxyméthylamidons sodiques faiblement substitués ou glycolate sodique d'amidon, sont des dérivés faiblement substitués et réticulés d'amidon de pomme de terre qui peuvent être utilisés en compression directe ou après granulation humide.

Lot D3 et D'3 : La Croscarmellose sodique utilisée dans cet exemple respectivement à 0.5 et 1% pour les lots D3 et D'3 est le Vivasol® (Pima, Allemagne, Lot 0404041) ou carboxyméthylcellulose sodique réticulée. C'est un excipient classiquement utilisé dans des formes pharmaceutiques orales en tant que désintégrant dans des comprimés ou des granules.

**Tableau 10: Définition et composition des lots en fonction de la qualité et de la quantité de polymère utilisées.**

| LOT | Dénomination du Lot | Quantité de polymère |
|---|---|---|
| XNPT 4610 | D1 | 0,5% de polyvinylpyrrolidone réticulée (10g) |
| XNPT 4579 | D1' | 1% de polyvinylpyrrolidone réticulée (20g) |
| XNPT 4580 | D2 | 0.5% de carboxyméthylamidon sodique (10g) |
| XNPT 4581 | D2' | 0,5% de carboxyméthylamidon sodique (20g) |
| XNPT 4582 | D3 | 0,5% de croscarmellose sodique (10g) |
| XNPT 4609 | D3' | 1% de croscamellose sodique (20g) |

**Tableau 11 : Temps de désagrégation des comprimés des lots de comprimés de microgranules contenant un agent désintégrant pour deux valeurs de contraintes de rupture,**

| | | **Contrainte à la rupture d'environ 0,3MPa** | **Contrainte à la rupture d'environ 0,5MPa** |
|---|---|---|---|
| 0,5% polyvinylpyrrolidone reticulée | D1 | 19 secondes | 1 minute |
| 1% polyvinylpyrrolidone réticulée | D1' | 26 secondes | 2 minutes 13 secondes |
| 0,5% carboxyméthylamidon sodique | D2 | 21 secondes | 1 minute et 46 secondes |
| 1 % carboxyméthylamidon sodique | D2' | 32 secondes | 2 minutes et 8 secondes |
| 0,5% croscarmellose sodique | D3 | 34 secondes | 3 minutes et 5 secondes |
| 1% croscarmellose sodique | D3' | 27 secondes | 1 minute et 45 secondes |

Cet exemple montre tout d'abord que les comprimés à base de désintégrants obtenus selon les caractéristiques de la présente invention présentent bien une capacité à se désagréger rapidement (en moins de 3 minutes) dans un milieu aqueux et ce pour des quantités de polymère très faibles.

Par ailleurs, cet exemple montre l'influence de la force de compression sur la vitesse de désagrégation de ces comprimés. Ainsi, très logiquement, moins la force de compression (et de rupture) est élevée et plus les comprimés obtenus se désagrègent rapidement. En effet, comme les microgranules poudrés sont moins comprimés, le réseau poreux sera donc plus large et laissera plus facilement passer le milieu liquide, favorisant la désagrégation rapide de la structure.

En revanche, cet exemple montre que l'influence de la nature du polymère désintégrant employé sur le temps de désintégration des comprimés est minime, voire nulle.

## Revendications

1. Comprimés faiblement dosés obtenus par compression, directe de microgranules qui sont essentiellement constitués d'un support neutre, recouvert d'une couche polymérique comprenant au moins un polymère pharmaceutiquement acceptable et permettant la libération modifiée de principes actifs en milieu aqueux, sur laquelle est appliquée une couche active comportant au moins un principe actif.

2. Comprimés selon la revendication 1, **caractérisés en ce que** ladite couche polymèrique comporte en outre au moins un agent liant pharmaceutiquement acceptable.

3. Comprimés selon la revendication 1 ou 2, **caractérisés en ce que** la quantité totale de polymère de ladite couche polymerique représente entre 1 est 100 % en masse de la masse du support neutre, de préférence entre 1 et 50 % en masse de la masse du support neutre.

4. Comprimés selon l'une quelconque des revendications 1 à 3, **caractérisés en ce que** ledit polymère est choisi parmi les polymères à libération prolongée et les polymères désintégrants.

5. Comprimés selon la revendication 4, **caractérisés en ce que** lesdites polymères désintégrants sont choisis parmi les dérivés de la polyvinylpyrrolidone, les dérivés de l'amidon, les sels de calcium et de magnésium, les alginates et les dérivés de la cellulose, ainsi que leurs mélanges,

6. Comprimes selon la revendication 5, **caractérisés en ce que** lesdits polymères désintégrants sont choisis parmi la crospovidone, la povidone, la carboxyméthylcellulose sodique, la croscarmellose sodique, la méthylcellulose, l'hydroxypropylcellulose faiblement substituée, le carboxyméthylamidon sodique, l'amidon réticulé, ainsi que leurs mélanges.

7. Comprimés selon la revendication 4, **caractérisés en ce que** lesdits polymères à libération prolongée sont choisis parmi des polymères de nature hydrophile à propriétés gélifiantes, de préférence de viscosité supérieure à 1000 m Pa.s, mesurée dans une solution aqueuse à 2% m/m à 20°C.

8. Comprimés selon la revendication 7, **caractérisés en ce que** lesdits polymères à libération prolongée sont choisis parmi les polymères dérivés de la cellulose, les polysaccharides naturels ou naturels modifiés tels que les gommes, les galactomananes, les glucomananes, les succinoglycanes ou les scléroglucanes, les carbomères et les poly(oxyde d'éthylène), ainsi que leurs mélange.

9. Comprimés selon la revendication 8. **caractérisés en ce que** lesdits polymères dérivés de la Cellulose sont des éthers de cellulose de moyenne à forte viscosité choisis parmi l'hydroxyéthylcellulose, l'hydroxypropylcellulose, et l'hydroxypropylméthylcellulose, ainsi que leurs mélanges.

10. Comprimés selon la revendication 8, **caractérisés en ce que** lesdits carbomères appartiennent au groupe comprenant le Carbopol® 971 P, le Carbopol® 974 P et le Carbopol® 934 P.

11. Comprimés selon la revendication 8, **caractérisés en ce que** lesdites gommes sont choisies parmi l'acide alginique, les alginates, l'agar-agar, les carraghénanes, la gomme de caroube, la gomme guar, la gomme adragante, la gomme arabique, la gomme de cassia, la gomme xanthane, la gomme karaya, la gomme tara, la gomme tragacanthe et la gomme galante, ainsi que leurs mélanges,

12. Comprimés selon la revendication 4, **caractérisés en ce que** lesdits polymères à libération prolongée sont choisis parmi les polymères et copolyméres dérivés de l'acide méthacrylique insolubles dans l'eau quelque soit le pH, ainsi que leurs mélanges,

13. Comprimes selon la revendication 12, **caractérisés en ce que** lesdits polymères à libération prolongée sont choisis parmi les chlorures de poly(éthyl acrylate, méthyl méthacrylate, triméthylamonioéthyl méthacrylate).

14. Comprimés selon la revendication 4, **caractérisés en ce que** lesdits polymères à libération prolongée sont choisis parmi les dérivés cellulosique insolubles dans l'eau, ainsi que leurs mélanges.

15. Comprimés selon la revendication 14, **caractérisés en ce que** lesdits polymères à libération plongée sont choisis parmi l'éthylcellulose et l'acétate de cellulose, ainsi que leurs mélanges.

16. Comprimés selon la revendication 4, **caractérisés en ce que** lesdits polymères à libération prolongée sont choisis parmi les polymères muco-adhésifs comme la carboxyméthylcellulose sodique, les carbomères, l'alginate de sodium, l'hydroxyéthylcellulose, l'hydroxypropylcellulose, l'hydroxypropylméthylcellulose, la gélatine, la gomme guar, les poly(oxyde d'éthylène), la dextrine ou le chitosane

17. Comprimés selon l'une quelconque des revendications 1 à 16, **caractérisés en ce que** ladite couche polymérique comprend en outre une cire ou un de ses dérivés, un dérivé d'acide gras du glycérol, ou un mélange de ceux-ci.

18. Comprimés selon la revendications 17, **caractérisés en ce que** la cire est choisi parmi la cire d'abeille naturelle on purifiée.

19. Comprimés selon la revendication 17, **caractérisés en ce que** le dérivé d'acide gras du glycérol est choisi parmi le glycérol monostéarate, le glycérol monooléate, le glycérol palmitostéarate et les mélanges de glycérides et d'esters d'acides gras du
polyéthylène glycol tels que ceux appartenant à la famille des lauroyl macrogolglycerides.

20. Comprimés selon l'une quelconque des revendications 1 19, **caractérisés en ce que** ladite couche active comporte en outre au moins un agent liant pharmaceutiquement acceptable,

21. Comprimés selon l'une quelconque des revendications 1 à 20, **caractérisés en ce que** ledit support neutre est une microsphère constituée de saccharose et d'amidon de maïs, de taille comprise entre 50 et 3000 µm, de préférence entre 100 et 1000 µm et plus préférentiellement encore entre 100 et 500µm.

22. Comprimés selon l'une quelconque des revendications 1 à 21, **caractérisés en ce qu'**ils contiennent en outre un lubrifiant dans une quantité inférieure à 5% en masse par rapport à la masse totale du comprimé.

23. Comprimés selon l'une quelconque des revendications 1 à 22, **caractérisés en ce qu'**ils sont en outre enrobés par une ou plusieurs couches d'agents de pelliculage.

24. Comprimés selon la revendication 23, **caractérisés en ce que** lesdits agents de pelliculage sont des agents de pelliculage gastro-résistants choisis parmi des polymères dérivés de l'acide méthacrylique, en particulier des copolyméres de l'acide méthacrylique, et des dérivés du polyvinylacétate tels que le polyvinylacétate phtalate, tels que les polymétahcrylique acide, éthyl acrylate, des dérivés de la cellulose tels que l'hydroxypropylméthyl cellulose phtalate, ainsi que leurs mélanges.

25. Comprimés selon l'une quelconque des revendications 1 à 24, **caractérisés en ce qu'**ils contiennent chacun moins de 50 mg, de préférence moins de 25 mg, encore plus préférentiellement moins de 10 ring de principe actif.

26. Comprimés selon l'une quelconque des revendications 1 à 25, **caractérisés en ce que** le principe actif est choisi parmi les hormones ou leurs dérivés, les principes actifs agissant sur le système nerveux central, les principes actifs agissant sur le système cardiovasculaire, les antibiotiques, les anti-viraux, les analgésiques et les anti-inflammatoires.

27. Comprimés selon la revendication 26, **caractérisées en ce que** lesdits principes actifs agissant sur le système nerveux central sont choisis parmi les anti-épileptiques, les anti-parkinsonniens, les psycho-Stimulants, les psychotropes, les antidépresseurs, les anxiolytiques et les anti-psychotiques.

28. Comprimés selon la revendication 26, **caractérisés en ce que** lesdits principes actifs agissant sur le système cardiovasculaire sont choisis parmi les anti-hypertenseurs, les anti-thrombotiques, les anti-agrégants et les hypocholestérolémiants.

29. Comprimés selon l'une quelconque des revendications 1 à 28, dans lesquels le principe actif est réparti de façon homogéne.

30. Comprimés selon l'une quelconque des revendications 1 à 29, caractérisés en qu'ils se présentent sous forme sécable.

31. Procédé de préparation des comprimés selon l'une quelconque des revendications 1 à 30, comprenant les étapes suivantes :
le support neutre est préalablement humidifié à l'aide d'une solution de mouillage contenant éventuellement un agent liant ;
- le polymère est ensuite appliqué à la surface du support neutre par poudrage ;
- une solution de montage comprenant le principe actif et éventuellement un agent liant, est pulvérisée sur la surface de la couche polymérique ;
- les microgranules ainsi obtenus sont ensuite sèchés, puis directement comprimés;
- le comprimé ainsi obtenu est éventuellement enrobé d'une ou plusieurs couches d'agent de pelliculage.

32. Procédé de préparation des comprimés selon la revendication 31, caractérisé en ce la compression s'effectue à l'aide de moins de 5% en masse de lubrifiant par rapport à la masse totale des comprimés.

33. Excipient fonctionalisé constitué d'un support neutre recouvert d'une couche polymérique dépourvue de principe actif comprenant au moins un polymère pharmaceutiquement acceptable et permettant la libération modifiée de principes actifs en milieu aqueux.

34. Microgranule constitué d'un support neutre recouvert d'une couche polymérique comprenant au moins un polymère pharmaceutiquement acceptable et permettant la libération modifiée de principes actifs en milieu aqueux, sur laquelle est appliquée une couche active comportant au moins un principe actif.

## Claims

1. Low-dose tablets obtained by the direct compression of microgranules which are essentially comprised of a neutral support, coated with a polymeric layer comprising at least one pharmaceutically acceptable polymer and allowing the modified release of the active principles in an aqueous medium, to which is applied an active layer containing at least one active principle.

2. Tablets according to claim 1, **characterized in that** the said polymeric layer contains in addition at least one pharmaceutically acceptable binding agent.

3. Tablets according to the claim 1 or 2, **characterized in that** the total quantity of the polymer of the said polymeric layer represents between 1 % and 100 % by weight of the weight of the neutral support, preferably between 1 % and 50 % by weight of the weight of the neutral support.

4. Tablets according to any of the claims 1 to 3, **characterized in that** the said polymer is selected among the extended-release polymers and the disintegrating polymers.

5. Tablets according to claim 4, **characterized in that** the said disintegrating polymers are selected among the polyvinylpyrrolidone derivatives, the starch derivatives, the calcium and magnesium salts, the alginates and the cellulose derivatives, as well as the mixtures thereof.

6. Tablets according to claim 5, **characterized in that** the said disintegrating polymers are selected among crospovidone, povidone, sodium carboxymethylcellulose, sodium croscarmellose, methylcellulose, low-substituted hydroxypropylcellulose, sodium carboxymethyl starch and crosslinked starch, as well as the mixtures thereof.

7. Tablets according to claim 4, **characterized in that** the said extended-release polymers are selected among the polymers of hydrophilic nature with gelling properties, preferably of a viscosity higher than 1000 mPa.s, measured in a 2% w/w aqueous solution at 20 °C.

8. Tablets according to claim 7, **characterized in that** the said extended-release polymers are selected among the polymers derived from cellulose, the natural or modified natural polysaccharides such as the gums, the galactomannans, the glucomannans, the succinoglycans, the scleroglucans, the carbomers and the poly(ethylene oxides), as well as the mixtures thereof.

9. Tablets according to claim 8, **characterized in that** the said polymers derived from cellulose are cellulose ethers of medium to high viscosity chosen among hydroxyethylcellulose, hydroxypropylcellulose, and hydroxypropylmethylcellulose, as well as the mixtures thereof.

10. Tablets according to claim 8, **characterized in that** the said carbomers belong to the group comprising Carbopol^{®} 971 P, Carbopol^{®} 974 P and Carbopol^{®} 934 P.

11. Tablets according to claim 8, **characterized in that** the said gums are selected among alginic acid, the alginates, agar-agar, the carrageenans, carob gum, gum guar, gum tragacanth, gum arabic, cassia gum, xanthan gum, gum karaya, tara gum and gellan gum, as well as the mixtures thereof.

12. Tablets according to claim 4, **characterized in that** the said extended-release polymers are selected among the polymers and copolymers derived from methacrylic acid insoluble in water regardless of pH, as well as the mixtures thereof.

13. Tablets according to claim 12, **characterized in that** the said extended-release polymers are selected among the poly(ethyl acrylate, methyl methacrylate, trimethylamonioethyl methacrylate) chlorides.

14. Tablets according to claim 4, **characterized in that** the said extended-release polymers are selected among the cellulose derivatives insoluble in water, as well as the mixtures thereof.

15. Tablets according to claim 14, **characterized in that** the said extended-release polymers are selected among ethylcellulose and cellulose acetate, as well as the mixtures thereof.

16. Tablets according to claim 4, **characterized in that** the said extended-release polymers are selected among the mucoadhesive polymers such as sodium carboxymethylcellulose, the carbomers, sodium alginate, hydroxyethylcellulose, hydroxypropylcellulose, hydroxypropylmethylcellulose, gelatin, guar gum, poly(ethylene oxide), dextrin and chitosan.

17. Tablets according to any of the claims 1 to 16, **characterized in that** the said polymeric layer comprises in addition a wax or a derivative thereof, a glycerol fatty acid derivative, or a mixture thereof.

18. Tablets according to claim 17, **characterized in that** the wax is selected among natural or purified beeswax.

19. Tablets according to claim 17, **characterized in that** the glycerol fatty acid derivative is selected among glycerol monostearate, glycerol monooleate, glycerol palmitostearate, and the mixtures of the fatty acid esters and glycerides of polyethylene glycol, such as those belonging to the lauroyl macrogolglycerides family.

20. Tablets according to any of the claims 1 to 19, **characterized in that** the said active layer contains in addition at least one pharmaceutically acceptable binding agent.

21. Tablets according to any of the claims 1 to 20, **characterized in that** the said neutral support is a microsphere comprised of sucrose and of corn starch, of a size between 50 µm and 3000 µm, preferably between 100 µm and 1000 µm, and still more preferentially between 100 µm and 500 µm.

22. Tablets according to any of the claims 1 to 21, **characterized in that** they contain in addition a lubricant in a quantity less than 5 % by weight relative to the total weight of the tablet.

23. Tablets according to any of the claims 1 to 22, **characterized in that** in addition they are coated by one or more layers of film-coating agents.

24. Tablets according to claim 23, **characterized in that** the said film-coating agents are gastroresistant film-coating agents chosen among the polymers derived from methacrylic acid in particular from copolymers of methacrylic acid, and from derivatives of polyvinyl acetate such as polyvinyl acetate phthalate and polymethacrylic acid, from ethyl acrylate, from derivatives of cellulose such as hydroxypropylmethyl cellulose phthalate, as well as the mixtures thereof.

25. Tablets according to any of the claims 1 to 24, **characterized in that** each contains less than 50 mg, preferably less than 25 mg, even more preferentially less than 10 mg of the active principle.

26. Tablets according to any of the claims 1 to 25, **characterized in that** the active principle is selected among the hormones or the derivatives thereof, the active principles acting on the central nervous system, the active principles acting on the cardiovascular system, the antibiotics, the antivirals, the analgesics and the anti-inflammatories.

27. Tablets according to claim 26, **characterized in that** the said active principles acting on the central nervous system are selected among the anti-epileptics, the anti-Parkinson's drugs, the psychostimulants, the psychotropics, the antidepressants, the anxiolytics and the antipsychotics.

28. Tablets according to claim 26, **characterized in that** the said active principles acting on the cardiovascular system are selected among the antihypertensives, the antithrombotics, the anti-aggregating agents and the cholesterol-lowering agents.

29. Tablets according to any of the claims 1 to 28, in which the active principle is distributed homogeneously.

30. Tablets according to any of the claims 1 to 29, **characterized in that** they are provided in scored form.

31. A method of preparation of the tablets according to any of claims 1 to 30, comprising the following steps:
- the neutral support is previously moistened using a wetting solution possibly containing a binding agent;
- the polymer is then applied to the surface of the neutral support by powdering;
- a layering solution comprising the active principle and possibly a binding agent are sprayed onto the surface of the polymeric layer;
- the microgranules thus obtained are then dried, then directly compressed;
- the tablet thus obtained is possibly coated with one or more layers of a film-coating agent.

32. A method of preparation of the tablets according to claim 31, **characterized in that** said compression is carried out using a lubricant at less than 5 % by weight relative to the total weight of the tablets.

33. A functionalized excipient comprised of a neutral support coated with a polymeric layer comprising at least one pharmaceutically acceptable polymer and allowing the modified release of the active principles in an aqueous medium.

34. A microgranule comprised of a neutral support coated with a polymeric layer comprising at least one pharmaceutically acceptable polymer and allowing the modified release of the active principles in an aqueous medium, to which is applied an active layer containing at least one active principle.

## Patentansprüche

1. Gering dosierte Tabletten, erhalten durch direkte Verdichtung von Mikrokörnchen, die im Wesentlichen aus einem neutralen Träger, überzogen von einer polymeren Schicht, welche mindestens ein pharmazeutisch verträgliches Polymer umfasst und die modifizierte Freisetzung von Wirkstoffen in wässrigem Milieu erlaubt, auf welche eine aktive Schicht, welche mindestens einen Wirkstoff umfasst, aufgebracht ist, bestehen.

2. Tabletten nach Anspruch 1, **dadurch gekennzeichnet, dass** die polymere Schicht außerdem mindestens ein pharmazeutisch verträgliches Bindemittel umfasst.

3. Tabletten nach Anspruch 1 oder 2, **dadurch gekennzeichnet, dass** die Gesamtmenge von Polymer der polymeren Schicht zwischen 1 und 100 Masse-% der Masse des neutralen Trägers, vorzugsweise zwischen 1 und 50 Masse-% der Masse des neutralen Trägers ausmacht.

4. Tabletten nach einem der Ansprüche 1 bis 3, **dadurch gekennzeichnet, dass** das Polymer unter den Polymeren mit zeitlich ausgedehnter Freisetzung und den zerfallenden Polymeren ausgewählt ist.

5. Tabletten nach Anspruch 4, **dadurch gekennzeichnet, dass** die zerfallenden Polymere unter den Derivaten von Polyvinylpyrrolidon, den Stärkederivaten, den Calcium- und Magnesiumsalzen, den Alginaten und den Cellulosederivaten wie auch deren Mischungen ausgewählt sind.

6. Tabletten nach Anspruch 5, **dadurch gekennzeichnet, dass** die zerfallenden Polymere unter Crospovidon, Povidon, Natriumcarboxymethylcellulose, Natriumcroscarmellose, Methylcellulose, gering substituierter Hydroxypropylcellulose, Natriumcarboxymethylstärke, vernetzter Stärke wie auch deren Mischungen ausgewählt sind.

7. Tabletten nach Anspruch 4, **dadurch gekennzeichnet, dass** die Polymere mit zeitlich ausgedehnter Freisetzung unter Polymeren von hydrophiler Natur mit gelierenden Eigenschaften, vorzugsweise mit einer Viskosität über 1000 mPa.s, gemessen in einer 2%-igen (Masse/Masse) wässrigen Lösung bei 20°C, ausgewählt sind.

8. Tabletten nach Anspruch 7, **dadurch gekennzeichnet, dass** die Polymere mit zeitlich ausgedehnter Freisetzung unter den von Cellulose abgeleiteten Polymeren, den natürlichen oder modifizierten natürlichen Polysacchariden, wie den Gummis, den Galactomannanen, den Glucomannanen, den Succinoglycanen oder den Skleroglucanen, den Carbomeren und den Poly(ethylenoxid)en wie auch deren Mischungen ausgewählt sind.

9. Tabletten nach Anspruch 8, **dadurch gekennzeichnet, dass** die von Cellulose abgeleiteten Polymere Celluloseether von mittleren bis hoher Viskosität, ausgewählt unter Hydroxyethylcellulose, Hydroxypropylcellulose und Hydroxypropylmethylcellulose wie auch deren Mischungen, sind.

10. Tabletten nach Anspruch 8, **dadurch gekennzeichnet, dass** die Carbomere zu der Gruppe, welche Carbopol® 971 P, Carbopol® 974 P und Carbopol® 934 P umfasst, gehören.

11. Tabletten nach Anspruch 8, **dadurch gekennzeichnet, dass** die Gummis unter Alginsäure, den Alginaten, Agar-Agar, den Carragheenen, Johannisbrotgummi, Guar Gum, Tragantgummi, Gummi arabicum, Cassia-Gummi, Xanthangummi, Karaya-Gummi, Tara-Gummi, Tragantgummi und erstarrendem Gummi wie auch deren Mischungen ausgewählt sind.

12. Tabletten nach Anspruch 4, **dadurch gekennzeichnet, dass** die Polymere mit zeitlich ausgedehnter Freisetzung unter den in Wasser unabhängig von dessen pH unlöslichen, von Methacrylsäure abgeleiteten Polymeren und Copolymeren wie auch deren Mischungen ausgewählt sind.

13. Tabletten nach Anspruch 12, **dadurch gekennzeichnet, dass** die Polymere mit zeitlich ausgedehnter Freisetzung unter den Poly(ethylacrylat, methylmethacrylat, trimethylammoniumethylmethacrylat)-chloriden ausgewählt sind.

14. Tabletten nach Anspruch 4, **dadurch gekennzeichnet, dass** die Polymere mit zeitlich ausgedehnter Freisetzung unter den in Wasser unlöslichen Cellulosederivaten wie auch deren Mischungen ausgewählt sind.

15. Tabletten nach Anspruch 14, **dadurch gekennzeichnet, dass** die Polymere mit zeitlich ausgedehnter Freisetzung unter Ethylcellulose und Celluloseacetat wie auch deren Mischungen ausgewählt sind.

16. Tabletten nach Anspruch 4, **dadurch gekennzeichnet, dass** die Polymere mit zeitlich ausgedehnter Freisetzung unter den mukoadhäsiven Polymeren, wie Natriumcarboxymethylcellulose, den Carbomeren, Natriumalginat, Hydroxyethylcellulose, Hydroxypropylcellulose, Hydroxypropylmethylcellulose, Gelatine, Guar Gum, den Poly(ethylenoxid)en, Dextrin oder Chitosan, ausgewählt sind.

17. Tabletten nach einem der Ansprüche 1 bis 16, **dadurch gekennzeichnet, dass** die polymere Schicht des Weiteren ein Wachs oder eines von dessen Derivaten, ein Fettsäurederivat von Glycerol oder eine Mischung von jenen umfasst.

18. Tabletten nach Anspruch 17, **dadurch gekennzeichnet, dass** das Wachs unter natürlichem oder gereinigtem Bienenwachs ausgewählt ist.

19. Tabletten nach Anspruch 17, **dadurch gekennzeichnet, dass** das Fettsäurederivat von Glycerol unter Glycerolmonostearat, Glycerolmonooleat, Glycerolpalmitostearat und den Mischungen von Glyceriden und Polyethylenglycol-fettsäureestern wie jenen, die zu der Familie der Lauroylmacrogolglyceride gehören, ausgewählt ist.

20. Tabletten nach einem der Ansprüche 1 bis 19, **dadurch gekennzeichnet, dass** die aktive Schicht außerdem mindestens ein pharmazeutisch verträgliches Bindemittel umfasst.

21. Tabletten nach einem der Ansprüche 1 bis 20, **dadurch gekennzeichnet, dass** der neutrale Träger eine aus Saccharose und Maisstärke gebildete Mikrosphäre mit einer Größe zwischen 50 und 3000 µm, vorzugsweise zwischen 100 und 1000 µm und noch mehr bevorzugt zwischen 100 und 500 µm ist.

22. Tabletten nach einem der Ansprüche 1 bis 21, **dadurch gekennzeichnet, dass** sie außerdem ein Gleitmittel in einer Menge unter 5 Masse-% bezogen auf die gesamte Masse der Tablette enthalten.

23. Tabletten nach einem der Ansprüche 1 bis 22, **dadurch gekennzeichnet, dass** sie außerdem von einer oder mehreren Schichten von Überzugsmitteln umhüllt sind.

24. Tabletten nach Anspruch 23, **dadurch gekennzeichnet, dass** die Überzugsmittel magensaftresistente Überzugsmittel sind, die unter den von Methacrylsäure abgeleiteten Polymeren, insbesondere Copolymeren von Methacrylsäure, und Polyvinylacetat-Derivaten, wie Polyvinylacetatphthalat, wie auch den Polymethacrylsäuren, Ethylacrylat, Cellulosederivaten, wie Hydroxypropylmethylcellulosephthalat, wie auch deren Mischungen ausgewählt sind.

25. Tabletten nach einem der Ansprüche 1 bis 24, **dadurch gekennzeichnet, dass** sie jeweils weniger als 50 mg, vorzugsweise weniger als 25 mg, noch mehr bevorzugt weniger als 10 mg Wirkstoff enthalten.

26. Tabletten nach einem der Ansprüche 1 bis 25, **dadurch gekennzeichnet, dass** der Wirkstoff unter den Hormonen oder deren Derivaten, den auf das Zentralnervensystem wirkenden Wirkstoffen, den auf das Herz-Kreislauf-System wirkenden Wirkstoffen, den Antibiotika, den antiviralen Mitteln, den Analgetika und den entzündungshemmenden Mitteln ausgewählt ist.

27. Tabletten nach Anspruch 26, **dadurch gekennzeichnet, dass** die Wirkstoffe, die auf das Zentralnervensystem wirken, unter den Antiepileptika, den Antiparkinsonmitteln, den Psychostimulantien, den Psychopharmaka, den Antidepressiva, den Anxiolytika und den Antipsychotika ausgewählt sind.

28. Tabletten nach Anspruch 26, **dadurch gekennzeichnet, dass** die Wirkstoffe, die auf das Herz-Kreislauf-System wirken, unter den Antihypertensiva, den Antithrombotika, den Antiaggregationsmitteln und den Hypocholesterinämie-Mitteln ausgewählt sind.

29. Tabletten nach einem der Ansprüche 1 bis 28, in denen der Wirkstoff auf homogene Weise verteilt ist.

30. Tabletten nach einem der Ansprüche 1 bis 29, **dadurch gekennzeichnet, dass** sie in einer teilbaren Form vorliegen.

31. Verfahren zur Herstellung von Tabletten nach einem der Ansprüche 1 bis 30, welches die folgenden Schritte umfasst:
- der neutrale Träger wird vorab mit Hilfe einer Benetzungslösung, welche gegebenenfalls ein Bindemittel enthält, befeuchtet;
- das Polymer wird dann auf die Oberfläche des neutralen Trägers durch Stäuben aufgebracht;
- eine Zusammenfügungslösung, welche den Wirkstoff und gegebenenfalls ein Bindemittel umfasst, wird auf der Oberfläche der Polymerschicht zerstäubt;
- die so erhaltenen Mikrokörnchen werden dann getrocknet, dann direkt verdichtet;
- die so erhaltene Tablette wird gegebenenfalls mit einer oder mehreren Überzugsmittelschichten umhüllt.

32. Verfahren zur Herstellung von Tabletten nach Anspruch 31, **dadurch gekennzeichnet, dass** die Verdichtung mit Hilfe von weniger als 5 Masse-% Gleitmittel bezogen auf die gesamte Masse der Tabletten ausgeführt wird.

33. Funktionalisiertes Vehikel, welches aus einem neutralen Träger, welcher von einer wirkstofffreien polymeren Schicht, welche mindestens ein pharmazeutisch verträgliches Polymer umfasst und die modifizierte Freisetzung von Wirkstoffen in wässrigem Milieu erlaubt, bedeckt ist, gebildet wird.

34. Mikrokörnchen, welches aus einem neutralen Träger, welcher von einer polymeren Schicht, welche mindestens ein pharmazeutisch verträgliches Polymer umfasst und die modifizierte Freisetzung von Wirkstoffen in wässrigem Milieu erlaubt, bedeckt ist, auf welche eine aktive Schicht, welche mindestens einen Wirkstoff umfasst, aufgebracht ist, gebildet wird.
